(19) Europäisches Patentamt
European Patent Office
Office européen des brevets

(11) EP 1 304 377 B1

(12) **EUROPEAN PATENT SPECIFICATION**

(45) Date of publication and mention of the grant of the patent:
**22.10.2008 Bulletin 2008/43**

(21) Application number: **01984244.2**

(22) Date of filing: **18.07.2001**

(51) Int Cl.:
***C12N 15/12*** *(2006.01)* ***C12Q 1/68*** *(2006.01)*
***G01N 33/574*** *(2006.01)*

(86) International application number:
**PCT/JP2001/006201**

(87) International publication number:
**WO 2002/006484 (24.01.2002 Gazette 2002/04)**

(54) **METHOD OF DETECTING CANCER**

VERFAHREN ZUM NACHWEIS VON KREBS

PROCEDE DE DETECTION DE CANCER

(84) Designated Contracting States:
**AT BE CH CY DE DK ES FI FR GB GR IE IT LI LU MC NL PT SE TR**

(30) Priority: **19.07.2000 JP 2000219807**

(43) Date of publication of application:
**23.04.2003 Bulletin 2003/17**

(73) Proprietor: **TAKARA BIO INC.**
**Otsu-shi,**
**Shiga 520-2193 (JP)**

(72) Inventors:
- **TSUBOSA, Yasuhiro**
  **Kusatsu-shi,**
  **Shiga 525-0032 (JP)**
- **AOYAGI, Kazuhiko**
  **Tokyo 164-0011 (JP)**
- **SASAKI, Hiroki**
  **Tokyo 104-0045 (JP)**
- **TERADA, Masaaki**
  **Tokyo 177-0053 (JP)**
- **MINENO, Junichi**
  **Uji-shi,**
  **Kyoto 611-0002 (JP)**
- **ASADA, Kiyozo**
  **Koka-gun,**
  **Shiga 520-3333 (JP)**
- **KATO, Ikunoshin**
  **Uji-shi,**
  **Kyoto 611-0028 (JP)**

(74) Representative: **Vossius & Partner**
**Siebertstrasse 4**
**81675 München (DE)**

(56) References cited:
**WO-A-01/74405** **WO-A1-96/02674**

- **ROSS D T ET AL: "SYSTEMATIC VARIATION IN GENE EXPRESSION PATTERNS IN HUMAN CANCER CELL CEL LINES" NATURE GENETICS, NATURE AMERICA, NEW YORK, US, vol. 24, March 2000 (2000-03), pages 227-235, XP002933374 ISSN: 1061-4036**
- **CASKEY L S ET AL: "TOWARD A MOLECULAR CLASSIFICATION OF THE GLIOMAS: HISTOPATHOLOGY, MOLECULAR GENETICS, AND GENE EXPRESSION PROFILING" HISTOLOGY AND HISTOPATHOLOGY, MURCIA, ES, vol. 15, no. 3, July 2000 (2000-07), pages 971-981, XP009002966 ISSN: 0213-3911**
- **ZUKERBERG LAWRENCE R ET AL: "Expression of the retinoblastoma protein in low-grade B-cell lymphoma: Relationship to cyclin D1" BLOOD, vol. 88, no. 1, 1996, pages 268-276, XP002284577 ISSN: 0006-4971**
- **ANBAZHAGAN R ET AL: "CLASSIFICATION OF SMALL CELL LUNG CANCER AND PULMONARY CARCINOID BYGENE EXPRESSION PROFILES" CANCER RESEARCH, AMERICAN ASSOCIATION FOR CANCER RESEARCH, BALTIMORE, MD, US, vol. 59, 15 October 1999 (1999-10-15), pages 5119-5122, XP002901773 ISSN: 0008-5472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WANG T ET AL: "IDENTIFICATION OF GENES DIFFERENTIALLY OVER-EXPRESSED IN LUNG SQUAMOUS CELL CARCINOMA USING COMBINATION OF CDNA SUBTRACTION AND MICROARRAY ANALYSIS" ONCOGENE, BASINGSTOKE, HANTS, GB, vol. 19, no. 12, 16 March 2000 (2000-03-16), pages 1519-1528, XP000951444 ISSN: 0950-9232
- A.A. ALIZADEH ET AL.: 'Distinct types of diffuse large B-cell lymphoma identified by gene expression profiling' NATURE vol. 403, February 2000, pages 503 - 511, XP002948333
- T.R. GOLUB ET AL.: 'Molecular classification of cancer: Class discovery and class prediction by gene expression monitoring' SCIENCE vol. 286, 15 October 1999, pages 531 - 537, XP002948334
- O. ISOZAKI ET AL.: 'Expression of insulin-like growth factor binding proteins (IGFBPs) in human thyroid papillary cancer tissues' ENDOCRINE JOURNAL vol. 43, no. SUPPL., 1996, pages S97 - S98, XP002948335
- R.E. HEWITT ET AL.: 'The activation, expression and function of gelatinase A (MMP-2)' TRENDS IN GLYCOSCIENCE AND GLYCOTECHNOLOGY vol. 8, no. 39, 1996, pages 23 - 36, XP002948336
- H. WAKITA ET AL.: 'Human squamous-cell carcinoma cell line (DJM-1) cells synthesize P-cadherin molecules via an elevation of extracellular calcium: Calcium regulates P-cadherin-gene expression at the translational level via protein tyrosine phosphorylation' INT. J. CANCER vol. 73, no. 3, 1997, pages 432 - 439, XP002948337
- S.R. LAKHANI ET AL.: 'Malignant myoepithelioma (myoepithelial carcinoma) of the breast: a detailed cytokeratin study' J. CLIN. PATHOL. vol. 48, no. 2, 1995, pages 164 - 167, XP002948338
- T. ISHIKAWA ET AL.: 'Matrilysin is a marker for progression of colorectal cancer' YOKOHAMA MED. BULL. vol. 46, no. 3-4, 1995, pages 92 - 93, XP002948339
- K. KEYOMARSI ET AL.: 'Reductant cyclin overexpression and gene amplification in breast cancer cells' PROC. NATL. ACAD. SCI. USA vol. 90, no. 3, February 1993, pages 1112 - 1116, XP002948340
- R.L. HUUHTANEN ET AL.: 'Expression of cyclin A in soft tissue sarcomas correlates with tumor aggressiveness' CANCER RES. vol. 59, no. 12, 1999, pages 2885 - 2890, XP002948341
- V. YERLY-MOTTA ET AL.: 'Expression of cyclins and CDKs throughout murine carcinogenesis' CELL MOL. BIOL. vol. 45, no. 8, 1999, pages 1217 - 1228, XP002948342
- N. KUZUMAKI: 'Expression of oncogene products and tumor markers in human cancers' TANPAKUSHITSU, KAKUSAN, KOUSO (PROTEIN, NUCLEIC ACIDS, ENZYME) vol. 32, no. 13, 1987, pages 1572 - 1576, XP002948343

SEQUENCE LISTING

<110> Takara Shuzo Co,. Ltd.

<120> Method for detecting cancer

<130> 01-051-PCT

<150> JP 2000-219807
<151> 2000-07-19

<160> 13

<210>
<211> 20
<212> DNA
<213> Artificial Sequence

<220>
<223> Description of Artificial Sequence: A sequence for PCR primer to a mplify a portion of E2F-1 gene.

<400> 1
ggccgtcctc ccagcctgtt 20

<210> 2
<211> 20
<212> DNA
<213> Artificial Sequence

<220>
<223> Description of Artificial Sequence: A sequence for PCR primer to a mplify a portion of B2F-1 gene.

<400> 2
aggctcacca aagaggcctc 20

<210> 3
<211> 63
<212> DNA
<213> Artificial Sequence

<220>
<223> Description of Artificial Sequence: A sequence for primer for reve rse transcription

<400> 3

ggccagtgaa ttgtaatacg actcactata gggaggcggt tttttttttt tttttttttt 60
ttt 63

<210> 4
<211> 30
<212> DNA
<213> Artificial Sequence

<220>

<223> Description of Artificial Sequence: A sequence for PCR primer to a mplify a portion of type I cytoskeletal 14 keratin gene.

<400> 4
tatgagacag agttgaacct gcgcatgagt 30

<210> 5
<211> 30
<212> DNA
<213> Artificial Sequence
<220>
<223> Description of Artificial Sequence: A sequence for PCR primer to a mplify a portion of type I cytoskeletal 14 keratin gene.

<400> 5
atgaagctgt attgattgcc aggagggggt 30

<210> 6
<211> 30
<212> DNA
<213> Artificial Sequence
<220>
<223> Description of Artificial Sequence: A sequence for PCR primer to a mplify a portion of type II cytoskeletal 6 keratin gene.

<400> 6
ctggacgtgg agatcgccac ctaccgcaag 30

<210> 7
<211> 30
<212> DNA
<213> Artificial Sequence
<220>
<223> Description of Artificial Sequence: A sequence for PCR primer to a mplify a portion of type II cytoskeletal 6 keratin gene.

<400> 7
caggctttgt acatcatagg actagtcact 30

<210> 8
<211> 30
<212> DNA
<213> Artificial Sequence
<220>
<223> Description of Artificial Sequence: A sequence for PCR primer to a mplify a portion of type II cytoskeletal 5 keratin gene.

<400> 8
tccagcgtca aatttgtctc caccacctcc 30

<210> 9
<211> 30
<212> DNA
<213> Artificial Sequence
<220>
<223> Description of Artificial Sequence: A sequence for PCR primer to a mplify a portion of type II cytoskeletal 5 keratin gene.

<400> 9
atttgggatt ggggtgggga ttctgttttg 30

<210> 10
<211> 30
<212> DNA
<213> Artificial Sequence
<220>
<223> Description of Artificial Sequence: A sequence for PCR primer to a mplify a portion of gelatinase A gene.

<400> 10
ccaaagtctg aagagcgtga agtttggaag 30

<210> 11
<211> 30
<212> DNA
<213> Artificial Sequence
<220>
<223> Description of Artificial Sequence: A sequence for PCR primer to a mplify a portion of gelatinase A gene.

<400> 11
catacttgtt gacatttccc atgggaatcg 30

<210> 12
<211> 30
<212> DNA
<213> Artificial Sequence
<220>
<223> Description of Artificial Sequence: A sequence for PCR primer to a mplify a portion of type II cytoskeletal 7 keratin gene.

<400> 12
agttggaggc cgccattgcc gaggctgagg 30

<210> 13
<211> 30
<212> DNA
<213> Artificial Sequence
<220>
<223> Description of Artificial Sequence: A sequence for PCR primer to a mplify a portion of type II cytoskeletal 7 keratin gene.

<400> 13
tggaagctat tctgacatca ctttccagac 30

## Description

### TECHNICAL FIELD

The present invention relates to a method for selecting a marker gene useful for cancer classification, a method for classifying cancer using the gene and a method for detecting cancer.

### BACKGROUND ART

Recently, the presence of the mechanism of multiple-stage carcinogenesis in which a normal cell transforms to a cancer has been clarified [Fearon, E.R. et al., Cell, 61, 759-767 (1990); and Sugimura, T., Science, 258, 603-607 (1992)]. Concretely, in the canceration of a normal cell, accumulation of plural abnormalities in genes including DNA repair gene, tumor suppressor gene and oncogene is said to be required. Generally, it is thought that instability of the gene and inactivation of tumor suppressor gene are involved in the development of cancer. Additionally, it is thought that activation of oncogene and/or overexpression of growth factor are involved in progress and transformation to malignant cancer. Further, it is thought that genes encoding degrading enzymes for extracellular matrix molecules and genes encoding proteins for regulating mobility or adhesive property of cells are involved in metastasis and infiltration.
As described above, acceleration or suppression of expressions of many genes is involved in the development, growth and metastasis of cancer.
Presently, genes involved in development and progress of cancer and information regarding abnormalities of the genes have been increasing on the level of individual genes. However, the mechanism of carcinogenesis comprises multiple stages and would require accumulation of plural numbers of mutations. Therefore, in the judgment by a single gene or the judgment by a random combination of a small number of genes, there have not yet obtained practically sufficient definite diagnosis of cancer and prognostic judgment at present.
Indeed, in the present days, the diagnosis and the judgment of progressive stage or differentiation degree of cancer are, in most cases, carried out by pathological diagnosis. However, among carcinoma showing similar progressive stage and similar differentiation degree, one may be a case that will exhibit good prognosis, while the other is a malignant case that will exhibit early recurrence or metastasis. Also, some cases show sensitivity to an anticancer agent and irradiation, while others exhibit resistance against them. In the current circumstances, it is therefore impossible to distinguish those cases before treatment or at an early stage after surgery.
It has been known in the chemotherapy of cancer that efficacies of chemotherapeutic agents are different for every kind of cancer. For example, in the case of 5FU (5-fluorouracil) and CDDP (cisplatin), these may have almost no effects on gastric cancer, colon cancer and liver cancer, while they have effects on uterine cervix cancer at its early stage in most cases. Approximately 50% of esophageal cancer cases are cases showing sensitivity to 5FU and CDDP. Accordingly, if one can know the drug sensitivities for individual cases, the effects of chemotherapy can be more enhanced. It is thought that the diagnosis and the type classification of cancer on the gene level are effective for such purposes.
In order to carry out the type classification as described above on the gene level, it is required to search and identify genes which show alterations in expression levels specifically to a particular type of cancer tissue. However, since alterations in expression of a large number of genes are found in a cancer tissue in association with cell proliferation, it is difficult to find appropriate marker genes.
When a cancer can be classified quickly and simply by the progressive stage, the differentiation degree and the type of the cancer, unnecessary treatment can be avoided, to select an appropriate treatment method. Therefore, such a classification procedure can contribute to minimize the burden on a patient and to establish a plan of treatment suitable for an individual patient. In addition, the above classification is thought to lead to reduction in medicinal expenditure.

### DISCLOSURE OF INVENTION

A first object of the present invention is to provide a method of finding a gene by which more accurate results regarding the cancer classification can be obtained on the gene level. In addition, a second object of the present invention is to provide a method for classifying cancer utilizing the gene found by the above method. Further, a third object of the present invention is to provide a method for detecting cancer.
In order to achieve the above objects, the present inventors have found a method for selecting a gene which is useful for the classification of genes and the evaluation for degree of malignancy, without affecting the genes each of which expressions are altered specifically during cell proliferation. Further, they have constructed a method of classifying cancer and a method for detecting cancer, each method using the gene found by the above method. The present invention has been accomplished thereby.
Specifically, the present invention relates to:

[1] a method for selecting a gene used as an index of cancer classification, comprising the following steps of:

(1) determining expression levels in cancer samples to be tested for at least one of genes each of which expression is altered specifically during cell proliferation, and then comparing the determined expression levels with an expression level of the genes in a control sample, thereby evaluating alterations in expression levels of the genes, wherein the control sample is a normal tissue, or a cancer sample with low malignancy;
(2) classifying the cancer samples to be tested into plural numbers of types, based on alterations in expression levels of the genes evaluated in the above step (1) and pathological findings for the cancer samples to be tested; and
(3) examining alterations in expressions for plural numbers of genes in each of the cancer samples to be tested classified in the above step (2), to select a gene, wherein expression of the above gene is altered independently to genes each of which expression is altered specifically during cell proliferation and expression level of the above gene is specifically altered depending on every type of cancer samples to be tested;

wherein said genes each of which expression is altered specifically during cell proliferation are selected from the group consisting of CDC6 gene and E2F family genes;
[2] a method for classifying a cancer, characterized in that cancer is classified with expression levels of genes in a sample to be tested, the method comprising the following steps:

(a) determining expression levels of at least one of genes used as indices of cancer classification, wherein the genes are selected by the selection method of item [1] above, in the sample to be tested, and
(b) comparing the gene expression levels determined in the step (a) with expression levels of the same genes in a control sample, thereby classifying cancer for the sample to be tested; and

[3] a method for detecting cancer, comprising the following steps:

(1) determining expression levels of at least one of genes used as indices of cancer classification in a sample to be tested, wherein the genes are selected by the selection method of item [1] above, and
(2) comparing the expression levels of genes in the sample to be tested determined in the step (1) with expression levels of the genes in a control sample, thereby detecting cancer,

wherein expressions of nucleic acids corresponding to at least one of genes or expressions of polypeptides encoded by at least one of the genes used as indices of cancer classification are altered compared with a control sample is an index of the presence of cancer cells in the sample to be tested;
Also described herein are a kit usable for classification and/or detection of cancer, comprising primers and/or a probe which is usable for determining expression levels of at least 5 kinds of genes selected from genes of Group I given below, and/or expression levels of at least 5 kinds of genes selected from genes of Group II given below;
a kit usable for classification and/or detection of cancer, comprising antibodies capable of binding specifically to polypeptides encoded by at least 5 kinds of genes selected from genes of Group I given below, and/or antibodies capable of binding specifically to polypeptides encoded by at least 5 kinds of genes selected from genes of Group II given below; and
a DNA array usable for classification and/or detection of cancer, wherein at least 5 kinds of genes selected from genes of Group I given below or fragments thereof, and/or at least 5 kinds of genes selected from genes of Group II given below or fragments thereof are immobilized at each of defined regions on a support.

## BEST MODE FOR CARRYING OUT THE INVENTION

In the present specification, the term "gene used as an index of cancer classification of the present invention" (hereinafter also referred to as "a marker gene") may be any gene that is useful for classifying types of cancer, and refers to a gene which can be used as a marker for cancer having some given characteristics. More concretely, the term "gene used as an index of cancer classification of the present invention" is a gene independent from genes each of which expression is altered specifically during cell proliferation, wherein the gene is a gene of which expression level is specifically altered depending upon the progressive stage, the differentiation degree, the type of cancer and the like. The term "gene used as an index of cancer classification" mentioned above is a gene that can be used as an index for the diagnosis of cancer (distinguishing cancer tissues from normal tissues), the classification of types of cancer, or the evaluation of the malignancy of the cancer, wherein the gene is a gene of which expression level is altered depending upon the types or malignancy of cancer, i.e. a gene of which expression is significantly induced or suppressed.

1. Method for Selecting Gene Used as Index of Cancer Classification of the Present Invention

One of the features of the method for selecting a gene used as an index of cancer classification of the present invention resides in that the method comprises the following steps:

(1) determining expression levels in cancer samples to be tested for at least one of genes each of which expression is altered specifically during cell proliferation, and then comparing the determined expression levels with an expression level of the genes in a control sample, thereby evaluating alterations in expression levels of the genes, wherein the control sample is a normal tissue, or a cancer sample with low malignancy;
(2) classifying the cancer samples to be tested into plural numbers of types, based on alterations in expression levels of the genes evaluated in the above step (1) and pathological findings for the cancer samples to be tested; and
(3) examining alterations in expressions for plural numbers of genes in each of the cancer samples to be tested classified in the above step (2), to select a gene, wherein expression of the above gene is altered independently to genes each of which expression is altered specifically during cell proliferation and expression level of the above gene is specifically altered depending on every type of cancer samples to be tested,

wherein said genes each of which expression is altered specifically during cell proliferation are selected from the group consisting of CDC6 gene and E2F family genes.

According to the selection method of the present invention, since a gene used as an index for suitable classification depending upon the cancer types of the sample is provided, there is exhibited an excellent effect that more reliable information can be obtained as compared with those obtained by conventional genetic methods for detecting and diagnosing cancer. In addition, according to the selection method of present invention, there is provided a gene of which expression level is altered independently from the genes each of which expression is altered specifically during cell proliferation, and altered specifically for every type of cancer samples to be tested. Therefore, by using the gene obtained by the selection method of the present invention as an index for detecting and diagnosing cancer, the distinctions between cancer cell proliferation and normal cell proliferation can be facilitated, thereby exhibiting an excellent effect that the reliability for detecting and diagnosing cancer can be enhanced.

In the selection method of the present invention, first of all, the expression states of genes each of which expression levels is altered specifically during cell proliferation in cancer lesion tissue (cancer tissues) are determined, and the cancer tissues are classified based on the expression levels.

Concretely, the expression level of at least one of genes each of which expression is altered specifically during cell proliferation in cancer samples to be tested is compared with an expression level of the genes in a control sample, thereby evaluating alterations in expression levels of the genes [referred to as step (1)].

The term "cancer sample(s) to be tested" used herein includes cancer lesion tissues, samples derived from patients with cancer who are suspected to have cancer cells, and the like.

The above-mentioned cancer samples to be tested include, for instance, samples derived from biological samples such as blood, urine, faeces, and tissues enucleated by any surgical procedure. In the method for selecting a gene used as an index of cancer of the present invention, lesion tissues obtained by using biopsy forceps are preferably used, from the viewpoints of preoperative diagnosis.

The control sample to be used in the above-mentioned step (1) includes, for instance, normal parts and cancer tissues with poor malignancy in the tissues enucleated by a surgical procedure.

The "genes each of which expression is altered specifically during cell proliferation" mentioned above include, for instance, genes associated with cell cycle. For instance, there can be used those which are known to be specifically expressed in cells during the DNA synthetic phase (S phase). In the selection method of the present invention, there can be used, for instance, CDC6 gene or genes belonging to E2F family can be used, and E2F-1 gene can be especially preferably used.

The method for determining the expression levels of genes each of which expression levels is altered specifically during cell proliferation is not particularly limited, and includes, for instance, a method for determining the expression level using a transcription product (such as mRNA) or a translation product (such as polypeptide) of the gene as an index and the like. In the above-mentioned step (1), a method for determining expression level of the gene by using mRNA as an index for which various means has been developed for its analysis with the progress of the gene manipulation techniques is an effective method.

The method for determining the expression level of a gene using a transcription product especially mRNA as an index includes the dot blot hybridization method, the Northern hybridization method, the RT-PCR method, the subtraction method, the differential display method and the like.

In addition, the expression levels of genes can be determined by the DNA array (DNA chip)-based hybridization analysis.

The method for determining the expression level of a gene using a translation product as an index includes, for instance, conventional immunoassays using an antibody against the translation product, and the like.

Next, in the selection method of the present invention, cancer samples to be tested are classified into plural number of types, based on alterations in expression levels of the genes evaluated in the above-mentioned step (1) and the pathological findings for the cancer samples to be tested [referred to as step (2)].

Concretely, the classification of cancer is carried out by using alterations in the expression levels of the genes as indices, wherein the alterations are evaluated by comparing the expression levels of the "genes each of which expression level is altered specifically during cell proliferation" in cancer samples to be tested, for instance, cancer tissues, with the expression levels of the genes in a control sample, in the above-mentioned step (1). In the above-mentioned step (1), when the amounts of alterations (absolute value) in the expression levels of the "genes each of which expression is altered specifically during cell proliferation" are at least 2-folds, preferably 3- or more folds, and especially preferably 5- or more folds as compared with those in normal tissues, expression of the genes in the cancer tissue is significantly altered.

Concretely, the classification of cancer tissue is carried out by using alterations in the expression levels of the genes as indices, the alterations being evaluated by comparing the expression level of, for instance, E2F-1 gene with the expression level of the above E2F-1 gene in a control sample, wherein a tissue in which expression of E2F-1 gene is increased to a level of at least 2-folds, preferably 3- or more folds, and especially preferably 5- or more folds, as compared with the expression in a normal tissue, is defined as an E2F-1-positive tissue.

Next, the cancer samples to be tested, for instance, the above-mentioned cancer tissues, are classified based on their pathological findings, for instance, cellular morphology, states of infiltration to the peripheral tissues, sensitivity against a drug, states of metastasis into lymph nodes, and the like. Such pathological findings are very important in, for instance, selecting a method of treatment and the like. Therefore, according to the method for selecting a gene used as an index of cancer classification of the present invention, there is provided a means capable of performing the pathological classification as described above without any surgical treatments for, for instance, cancer in a patient before initiation of treatment.

Subsequently, alterations in expressions for plural numbers of genes in each of the cancer samples to be tested classified in the above-mentioned step (2) are examined, to select a gene of which expression is altered independently from genes each of which expression is altered specifically during cell proliferation and of which expression level is specifically altered depending on every type of cancer samples to be tested [referred to as step (3)].

The above-mentioned term "gene used as an index of cancer classification" can be selected by specifying genes having differential expression levels between the control sample and the cancer samples to be tested, for instance, by comparing the expression level of a gene product in a cell used as control with the expression level of a gene product in a cell derived from cancer tissue, and specifying ones having differences in the expression levels between both of the above cells.

The control sample in step (3) includes, for instance, normal tissues and cancer samples with poor malignancy.

The cancer samples with poor malignancy include, for instance, samples with poor malignancy, for example, those exhibiting no lymph node metastasis, wherein the samples exhibit no alterations in expressions of "genes each of which expression is altered specifically during cell proliferation" mentioned above.

The gene product mentioned above includes, for instance, mRNAs transcribed from genes, and proteins which are translation products.

In the selection of the genes used in the present invention, it is effective to use mRNA as an index for which various analytic procedures are now developed with the progress in the gene manipulation techniques.

The technique for determination of alterations in the gene expressions using mRNA as an index includes, for instance, the dot blot hybridization method, the Northern hybridization method, the RT-PCR method, the subtraction method, the differential display method and the like. Any one of these methods can be suitably selected to find the gene used in the present invention. Further, as to the method for simultaneously detecting alterations in expressions of a large number of genes of hundreds or thousands of genes, hybridization assay using a DNA array (DNA chip) has been known, and can be suitably used in the present invention.

The above-mentioned term "DNA array" as used herein refers to an array (chip) which comprises a support, and a gene or a DNA fragment derived from the gene immobilized thereto in a defined region, including, for instance, one called "DNA chip." Also, an array which comprises a support, and a gene or DNA fragment derived from the gene immobilized thereto at a high density, for instance, a density of 100 genes/$cm^2$ or more, may be also referred to as "DNA microarray."

The support of the DNA array may be any of those which can be used for hybridization. Usually, a glass slide, a silicon chip, a nitrocellulose membrane, a nylon membrane or the like may be used. In addition, the gene to be immobilized or its fragment to the support includes, but are not particularly limited to, for instance, genomic DNA libraries, cDNA libraries, or DNAs amplified by, for instance, PCR with these libraries as a template, or the like.

By using the DNA array described above, the amounts of various kinds of nucleic acid molecules contained in a nucleic acid sample can be simultaneously determined. In addition, there is an advantage such that the determination can be carried out even with a small amount of the nucleic acid sample. For instance, mRNA in the sample is labeled, or labeled cDNA is prepared by using mRNA as a template, and the labeled mRNA or cDNA is subjected to hybridization with the DNA array, so that mRNAs being expressed in the sample are simultaneously detected, whereby their expression levels can be determined.

In the present invention, the "gene used as an index of cancer classification" can be selected by using, for instance, a DNA array to which a nucleic acid corresponding to a human-derived gene or a fragment thereof is immobilized. Currently, DNA microarrays to which gene fragments that are suggested or suspected to be related to cancer or other physiological phenomena are commercially available (e.g., IntelliGene Human Cancer CHIP or IntelliGene Apoptosis CHIP, both manufactured by Takara Shuzo Co., Ltd.). By using these DNA microarrays, the genes which are used as indices can be obtained by performing the above-mentioned steps (1)-(3).

Genes each of which expression is altered specifically in each type can be found by determining expression levels of various genes in the cancer tissues classified into certain types as described above and comparing the expression levels with the expression level in a control tissue.

The method for determining the expression levels of genes is not particularly limited, and any of techniques for confirming alterations of the gene expressions mentioned above can be suitably used. Among all, the method using the DNA array is especially preferable because the expressions of a large number of genes can be simultaneously determined.

For instance, mRNA is prepared from each type of cancer tissues, and then reverse transcription is carried out with the resulting mRNA as a template. During this process, labeled cDNA can be obtained by using, for instance, any suitable labeled primers or labeled nucleotides.

As to the labeling substance used for labeling, there can be used substances such as radioisotopes, fluorescent substances, chemiluminescent substances and substances with fluophor, and the like. For instance, the fluorescent substance includes Cy2, FluorX, Cy3, Cy3.5, Cy5, Cy5.5, Cy7, fluorescein isothiocyanate (FITC), Texas Red, Rhodamine and the like. In addition, it is desired that samples to be tested (cancer samples to be tested in the present selection method) and a sample to be used as a control are each labeled with different fluorescent substances, using two or more fluorescent substances, from the viewpoint of enabling simultaneous detection. Here, labeling of the samples is carried out by labeling mRNA in the samples, cDNA derived from the mRNA, or nucleic acids produced by transcription or amplification from cDNA.

Next, the hybridization is carried out between the above-mentioned labeled cDNA and the DNA array to which a nucleic acid corresponding to a suitable gene or its fragment is immobilized. The hybridization may be performed according to any known processes under conditions that are appropriate for the DNA array and the labeled cDNA to be used. For instance, the hybridization can be performed under the conditions described in Molecular Cloning, A laboratory manual, 2nd ed., 9.52-9.55 (1989).

The hybridization between the nucleic acids derived from the samples and the DNA array is carried out, under the above-mentioned hybridization conditions. When much time is needed for the time period required for procedures from the collection of samples to the determination of expression levels of genes, the degradation of mRNA may take place due to actions of ribonuclease. In order to determine the difference in the gene expressions in the samples to be tested (i.e., cancer samples to be tested in the present selection method) and the gene expressions in a control sample, it is preferable that the mRNA levels in both of these samples are adjusted using a standard gene with relatively little alterations in expressions. Further, when competitive hybridization is carried out on a single DNA array using two fluorescent substances described below, more accurate data can be obtained by adjusting the differences in the intensities of the two fluorescent substances. As to the nucleic acid to be used for the purpose of the adjustment described above, there are included nucleic acids derived from samples from non-lesion sites; and nucleic acids which correspond to housekeeping genes [e.g., glyceraldehyde-3-phosphate dehydrogenase (GAPD) gene, cyclophilin gene, β-actin gene, α-tubulin gene, phospholipase A2 gene or the like]. The negative control to be used for confirming that the hybridization is not non-specific hybridization includes nucleic acids which have completely no relevance to the samples, for instance, plasmid pUC18 or the like.

Thereafter, by comparing the hybridization results of the samples to be tested (cancer samples to be tested in the present selection method) with those of the control sample, genes exhibiting differential expression levels in both samples can be detected. Concretely, a signal which is appropriate depending upon the method of labeling used is detected for the array which is subjected to hybridization with the nucleic acid sample labeled by the method as described above, whereby the expression levels in the samples to be tested (cancer samples to be tested in the present selection method) can be compared with the expression level in the control sample for each of the genes on the array. Preferably, when a multi-wavelength detection fluorescence analyzer capable of detecting plural labeling, e.g., two kinds of fluorescence, is used, the difference between the gene expression levels in the samples to be tested (cancer samples to be tested in the present selection method) and the gene expression level in a control sample can be compared by competitive hybridization on the same DNA array. For instance, samples derived from cancer lesion tissue are fluorescent-labeled with Cy5-dUTP, while the control nucleic acid samples are fluorescent-labeled with Cy3-dUTP. The DNA array-based hybridization is carried out by mixing the samples to be tested and the control sample in an equivolume, whereby the difference in the gene expression levels of the samples to be tested and the control sample can be detected as differences in the colors of signal and in the fluorescence intensities.

The method for detecting labeled nucleic acids may be properly selected depending upon the kinds of the labeling substances used. For instance, when Cy3 and Cy5 mentioned above are used as the labeling substances, Cy3 can be detected by scanning at a wavelength of 532 nm, and Cy5 can be detected by scanning at a wavelength of 635 nm. The intensity of labeling is used as an index for expression levels of genes.

The control sample in the determination of alterations in the expression levels of genes described above is not particularly limited. There can be used samples derived from normal tissues; those cancer samples which are thought to have poor malignancy among the above-listed cancer samples, for instance, those having low level of expression of E2F-1 gene and showing no or little metastasis into lymph nodes; and the like.

The genes thus obtained which have a significant difference in signal intensities are genes each of which expression is altered specifically for every type of cancer tissues. Some of the genes vary in parallel with, or in a negative correlation with, genes each of which expression is altered specifically during cell proliferation. It is highly likely that these genes may have alterations in their expression levels simply reflecting the cell proliferation in the sample, and may not necessarily be marker genes which are suitably used in the classification of cancer samples. The present invention is characterized by finding a marker gene useful for more accurate type classification of cancer and evaluation of degree of malignancy of cancer by finding genes each of which expression is altered specifically during cell proliferation besides these genes.

Further, the marker genes thus found are not particularly limited. Those having greater differences in expression levels as compared to those of other types of cancer samples are more desirably used as indices for classification.

By utilizing the method for selecting a gene used as an index of cancer classification of the present invention, a gene useful for type classification of cancer can be obtained. For instance, with regard to metastasis into lymph nodes of esophageal cancer, using the genes listed in Table 1 below as the marker genes, there can be evaluated whether or not samples to be tested have a high risk of metastasis into lymph nodes.

Table 1

| Genes | GenBank Accession # |
|---|---|
| insulin-like growth factor binding protein 2 (IGFBP2) | X16302 |
| BIGH3 | M77349 |
| insulin-like growth factor binding protein 6 (IGFBP6) | M62402 |
| gelatinase A (MMP-2) | M55593 |
| type II cytoskeletal 7 keratin (cytokeratin 7 (K7; CK 7)) | M13955 |
| desmoplakin I | M77830 |
| glutathione S-transferase A1 | M16594 |
| glutathione S-transferase Pi (GSTP1) | U12472 |
| collagenase-3 (MMP-13) | X75308 |
| type II cytoskeletal 5 keratin (cytokeratin 5 (K5; CK 5)) | M21389 |
| P-cadherin | X63629 |
| type I cytoskeletal 14 keratin (cytokeratin 14 (K14; CK 14)) | J00124 |
| type II cytoskeletal 6 keratin (cytokeratin 6B (CK 6B)) | L42610 |
| matrilysin (MMP-7) | X07819 |
| forkhead-like 7 | AF048693 |
| connective tissue growth factor (CTGF) | M92934 |
| growth hormone-dependent insulin-like growth factor-binding protein | M35878 |
| Rho8 protein | X95282 |

In addition, using each of the genes listed in the following Table 2 as the marker genes, there can be evaluated whether or not the sample to be tested has an especially high risk among the cancers having the risk of metastasis into lymph nodes.

Table 2

| Genes | GenBank Accession # |
|---|---|
| RBA/p48 | X74262 |
| cell division control protein 2 homolog (EC 2.7.1.-)(cdc2) | X05360 |
| replication factor C 38-kDa subunit (RFC38) | L07541 |
| apopain precursor | U13737 |
| xeroderma pigmentosum group C repair complementing protein p58/HHR23B | D21090 |
| cyclin G2 | U47414 |
| cyclin A | X51688 |
| apoptosis-related protein TFAR15 | AF022385 |
| TRKB tyrosine kinase receptor | U12140 |
| signal transducer and activator of transcription 1-alpha/beta (STAT1) | M97935 |
| K-ras oncogene | M54968 |
| retinoblastoma susceptibility | L41870 |
| BCL2/adenovirus E1B 19kD-interacting protein 1 (BNIP1) mRNA, complete cds | AF083957 |
| inhibitor of apoptosis protein 1 (HIAP-1) | U45878 |

2. Method for Classifying Cancer of the Present Invention

One of the features in the method for classifying cancer of the present invention resides in that the method comprises the following steps:

(a) determining expression levels of at least one of genes used as indices of cancer classification, wherein the genes are selected by the selection method of the present invention, in a sample to be tested, and
(b) comparing the gene expression levels determined in the step (a) with expression levels of the same genes in a control sample, thereby classifying cancer for the sample to be tested.

In the classification method of the present invention, a gene independent from genes each of which expression is altered specifically during cell proliferation, wherein expression level of the gene is altered specifically depending upon the progressive stage, the differentiation degree, the types of the cancer and the like, is used as an index of cancer classification. Therefore, there is exhibited an excellent effect that the classification can be carried out for, for instance, cancer in a patient before starting the treatment, based on the cell morphology, infiltration states into the peripheral tissues, sensitivity against drugs, or states of metastases into lymph nodes, or the like. Also, according to the classification method of cancer, more reliable and useful information can be provided conveniently and quickly during, for instance, the selection of a method of treatment.
According to the method for classifying cancer of the present invention, samples derived from individuals who are suspected to have cancer, especially esophageal cancer, can be classified.
In the method for classifying cancer of the present invention, first of all, expression levels of at least one of genes used as indices of cancer classification are determined, wherein the genes are selected by the selection method of the present invention, in a sample to be tested [referred to as step (a)].
The above-mentioned samples to be tested include, for instance, samples derived from biological samples such as blood, urine, faeces, and tissues enucleated by any surgical procedure, the samples being derived from an individual who is suspected to have cancer, especially esophageal cancer.
In the above-mentioned step (a), from the viewpoint of classifying cancer more accurately, it is preferable that expression levels of plural genes are determined. For instance, expression levels of at least 5 kinds of marker genes may be determined, without being particularly limited thereto.
The "genes used as indices of cancer classification" used in the classification method of the present invention may be any genes obtained by the selection method of the present invention. For instance, any one appropriately selected from those listed in Table 1 and/or Table 2 above can be used.
The expression level of the "genes used as indices of cancer classification" mentioned above is determined based on the level of mRNA transcribed from the gene or the level of a polypeptide translated from the gene.
The method for determining the level of mRNA includes hybridization methods and nucleic acid amplification method, and concretely, a known method such as the dot blot hybridization method, the Northern hybridization method or the RT-PCR method can be employed. The determination method, which is not particularly limited to, is preferably hybridization method using a DNA array, especially a DNA microarray, in the present invention, from the viewpoints of being capable of performing simultaneous determination and comparison of large numbers of gene expressions using a small amount of samples. The DNA array-based hybridization method can be performed according to the procedures described above.

The level of the polypeptide encoded by the above-mentioned gene can be determined by, for instance, enzyme immunoassay, fluorescence immunoassay, luminescent immunoassay or the like, using an antibody against the polypeptide or fragments thereof. Concretely, the level of the polypeptide can be determined by, for instance, conventional ELISA method using a labeled antibody or the like.

The above-mentioned antibody is not particularly limited, as long as it has an ability of specifically binding to the above-mentioned polypeptide, which may be either polyclonal antibody or monoclonal antibody. Further, the above-mentioned term "antibody" encompasses antibody fragments obtained by fragmentation of the above-mentioned antibody, and modified antibodies or derivatives thereof obtained by any known methods, including, for instance, humanized-antibodies, Fab-fragments, single-stranded antibodies and the like. The above-mentioned antibody can be readily prepared by immunizing an animal such as rabbit, rat or mouse using all or part of the above-mentioned polypeptide in accordance with the method described in, for instance, Current Protocols in Immunology, John E. Coligan eds., John Wiely & Sons, Inc. (1992). The antibody thus obtained may be purified and then treated with peptidase or the like to give antibody fragments. Alternatively, an antibody can be engineered. Further, the above-mentioned antibody may be subjected to various modifications so that detection can be facilitated in, for instance, enzyme immunoassay, fluorescence immunoassay, luminescent immunoassay or the like.

Next, the gene expression levels determined in the above-mentioned step (a) are compared with expression levels of the same genes in a control sample, thereby classifying cancer for the above-mentioned sample to be tested [referred to as step (b)].

In the above-mentioned step (b), the cancer existing in the sample to be tested can be classified by comparing the expression levels of the marker genes in the sample to be tested determined in the step (a) with expression levels of the marker gene in the control sample, and then analyzing the patterns in alterations of the expression levels. When the marker gene is selected as described in Section 1. above, the patterns in alterations of the expression levels of each gene have been clarified for every type of cancer, so that the samples to be tested can be classified in reference to such patterns. In other words, in the classification method of the present invention, the gene of which expression is altered specifically depending on the progressive stage, the differentiation degree, the type or the like of cancer is used as an index of cancer classification, wherein the gene is obtained by the selection method of the present invention. Therefore, it is apparent that each of the genes is associated with the type of cancer, and the like, so that the samples to be tested can be classified by referring to the alterations in the expression levels of the genes.

The above-mentioned control sample includes, for instance, samples derived from normal tissues.

Instead of performing the step (b), the alterations in the expression levels of the genes used as indices of cancer classification can be found by comparison with the expression level of a gene having little alterations in the expression levels in a sample to be tested, for instance, an expression level of the housekeeping gene mentioned above, without using any control sample.

Especially, in the method for classifying cancer of the present invention using a DNA microarray, there is an advantage in that alterations in the expressions of a large number of genes can be examined with a very small amount of sample. In the method for classifying cancer using a DNA microarray, since the cancer tissue existing in the cancer lesion site can be classified by collecting only a part of the lesion site with, for instance, an endoscope or other means, the method is very useful as an index for the diagnosis of cancer or the selection of the method of treatment.

Concretely, for instance, the risk of metastasis into lymph nodes on the esophageal cancer can be evaluated based on the patterns in alterations of these genes by comparing the expression levels of the genes listed in Table 1 above in a sample derived from an esophageal cancer tissue with those in a sample derived from a normal esophageal tissue.

Further, there can be evaluated whether or not the samples to be tested have a cancer with the highest risk of metastasis into lymph nodes among those with such a risk, based on the patterns in alterations of the genes obtained by comparing the expression levels of the genes listed in Table 2 in a sample to be tested with those in a sample derived from a normal esophageal tissue.

In the classification method of the present invention, in the case where expressions of some of the genes listed in Table 1 are decreased, while expressions of some of the genes listed in Table 2 are increased in samples to be tested, the samples to be tested can be classified as samples which are predicted to have a high risk of metastasis into lymph nodes.

The results analyzed and obtained as described above may be output to a printer, a display device or software packages such as a graphic software for display. The output may be especially advantageous when the results obtained by the detection method of the present invention are used for the diagnosis, the selection of method of treatment and the like.

3. Method for Detecting Cancer of the Present Invention

The "genes used as indices of cancer classification" obtained by the selection method of the present invention are also useful as indices for detecting cancer, particularly esophageal cancer. Therefore, the present invention also provides a method for detecting cancer, especially esophageal cancer. The method for detecting cancer may be encompassed by the scope of the present invention.

In the method for detecting cancer of the present invention, the "genes used as indices of cancer classification" are used as indices of cancer, wherein expression of the gene is altered independently from genes each of which expression is altered specifically during cell proliferation and expression level of the gene is specifically altered depending on every type of cancer. Therefore, there are exhibited excellent effects such that the distinction between proliferation of cancer and normal cells can be facilitated, and that the cancer can be detected in a higher reliability. In addition, since the alteration in the expression levels of the "genes used as indices of cancer classification" reflects the progressive stage, the malignancy degree and the type of cancer, especially esophageal cancer, there are exhibited some excellent effects that the progressive stage, the malignancy degree and the type of cancer can be judged at the same time as the detection of the gene.

Concretely, one of the great features of the method for detecting cancer of the present invention resides in that the method comprises examining expression of a nucleic acid corresponding to at least one of "genes used as indices of cancer classification" or expression of a polypeptide encoded by the genes, wherein the genes are obtained by the above-mentioned selection method in that of a sample to be tested and in a control sample, thereby detecting cancer, especially esophageal cancer, using a difference in expressions of the genes or polypeptide between the samples to be tested and the control sample as an index that the sample to be tested contains cancer cells.

Concretely, the method for detecting cancer of the present invention comprises the following steps:

(1) determining expression levels of at least one of genes used as indices of cancer classification in a sample to be tested, wherein the genes are selected by the selection method of the present invention, and
(2) comparing the expression levels of genes in the sample to be tested determined in the step (1) with expression levels of the genes in a control sample, thereby detecting cancer.

The samples to be tested in the method for detecting cancer of the present invention include, for instance, samples derived from biological samples such as blood, urine, faeces, and tissues enucleated by any surgical procedure.

The control sample includes samples derived from a normal individual or cells derived from non-lesion sites, namely normal tissues.

Instead of performing the step (2), the alterations in the expression levels of the "genes used as indices for detection of cancer" can be found by comparison of the expression levels of the genes used as indices for detection of cancer with the expression level of a gene having little alterations in the expression level in a sample to be tested, for instance, an expression level of the housekeeping gene described above, without using any control sample.

In the detection method of the present invention, expression of a nucleic acid corresponding to at least one of the "genes used as indices of cancer classification" or expression of a polypeptide encoded by each of the genes can be assayed by nucleic acid amplification method, hybridization method, enzyme immunoassay, fluorescence immunoassay, luminescent immunoassay and the like. In the present invention, when the expression of the nucleic acid is determined, it is more preferable that the expression of the gene is examined using the above-mentioned DNA array, from the viewpoints that a large number of the "genes used as indices of cancer classification" can be simultaneously examined, and that the time period required for detection can be shortened.

In the detection method of the present invention, alteration in expression of a nucleic acid corresponding to at least one of the "genes used as indices of cancer classification" or expression of a polypeptide encoded by each of the genes, in a sample to be tested in comparison with that in a control sample is an index showing that cancer cells exist in the above-mentioned sample to be tested, whereby cancer, especially esophageal cancer, can be detected, and the progressive stage, the malignancy degree, and the type of cancer, esophageal cancer, can be judged.

The results analyzed and obtained as described above may be output to a printer, a display or any software packages such as a graphic software for display. The output may be especially advantageous in the case where the results obtained by the detection method of the present invention are used for the diagnosis or the selection of method of treatment.

Kit Usable for Classification and/or Detection of Cancer

The kit described herein usable in the classification and/or detection of cancer includes a kit for examining alterations in the expression levels of the above-mentioned "genes used as indices of cancer classification" in a sample to be tested. In other words, the kit is used for determining the expression levels of at least one of the genes obtained by the method for selecting a gene used as an index of cancer classification of the present invention. It is preferable that the kit can determine the expression levels of at least 5 kinds of "genes used as indices of cancer classification", without being particularly limited thereto, from the viewpoint of more accurately classifying cancer.

The kit described herein is a kit capable of quantifying the level of mRNA transcribed from the above-mentioned "genes used as indices of cancer classification." One of the great features of the kit resides in that the kit comprises primers and/or a probe which is usable for determining the level of mRNA from the gene.

The kit described herein is based on the surprising findings made by the present inventors that the above-mentioned "genes used as indices of cancer classification," or more concretely, the genes listed in Tables 1 and 2, reflect the progressive stage, the malignancy degree and the type of cancer, especially esophageal cancer. According to the kit described herein, cancer, especially esophageal cancer, can be detected in high reliability by using oligonucleotides which are capable of specifically binding to, namely capable of hybridizing under stringent conditions, to nucleic acids corresponding to the above-described "genes used as indices of cancer classification," or more concretely each of the genes listed in Tables 1 and 2, or complementary strands (antisense strands) of the nucleic acids, and can be classified in accordance with the progressive stage, the malignancy degree and the type of cancer.

The above-mentioned primers include, for instance, any primers which are capable of specifically amplifying nucleic acid sequences derived from mRNA transcribed from the above-mentioned genes under reaction conditions used for any conventional nucleic acid amplification method, for instance, RT-PCR. For instance, primers can be designed and synthesized on the basis of the nucleotide sequences of those genes. Also, the length of the primers is, for instance, but not particularly limited to, 15 to 40 nucleotides in length and especially preferably 17 to 30 nucleotides in length.

In addition, it is desired that the kit described herein preferably comprises a pair of primers capable of specifically amplifying a nucleic acid sequence derived from mRNA transcribed from the above-mentioned genes.

When the amount of mRNA is determined by nucleic acid amplification method, or especially RT-PCR method, the amount of mRNA can be determined by competitive PCR method, TaqMan method [see, for instance, Linda G. Lee et al., Nucleic Acids Research 21, 3761-3766 (1993) or the like] and the like.

The above-mentioned probe or primers may be any of those which are capable of hybridizing to a nucleic acid corresponding to each of the above-mentioned "genes used as indices of cancer classification" (sense strand) or to a nucleic acid having a sequence complementary to each of the genes (antisense strand) under stringent conditions. The term "stringent conditions" referred to herein is not particularly limited, and includes, for instance, conditions in which the probe or primers are incubated overnight in a solution of 6 × SSC (wherein 1 × SSC means 0.15 M NaCl, 0.015 M sodium citrate, pH 7.0), 0.5% SDS, 5 × Denhardt's, 100 μg/ml denatured herring sperm DNA at a temperature of [*Tm* - 25°C of the above-mentioned primers and/or probe]. *Tm* of the probe can be obtained, for instance, by the following equation:

$$Tm = 81.5 - 16.6 (\log_{10}[Na^+]) + 0.41(\%G+C) - (600/N)$$

wherein N is a chain length of the probe, and %G+C is the contents of guanine and cytosine residues in the probe or primers.

When the chain length of the probe is shorter than 18 nucleotides in length, *Tm* can be estimated as the sum of the product of A+T (adenine + thymine) content by 2°C and the product of G + C content by 4°C, i.e.,

$$[(A + T) \times 2 + (G + C) \times 4].$$

The chain length of the above-mentioned probe is not particularly limited. It is desired that the probe has 15 nucleotides or more in length, and preferably 18 nucleotides or more in length, from the viewpoint of prevention of non-specific hybridization.

Furthermore, the kit described herein can contain, in addition to the above-mentioned primers and/or probe, reagents usable in nucleic acid amplification method, for instance, DNA polymerase (thermostable DNA polymerase which is suitable for PCR, especially LA-PCR), dNTP, $MgCl_2$, or substances for enhancing the polymerase reaction; reagents usable for detection; and the like.

<u>DNA Array</u>

The DNA array described herein usable for classification and/or detection of cancer is an array in which nucleic acids each corresponding to the above-mentioned genes used as indices of cancer classification or fragments thereof are immobilized at each of the defined regions on a support. In other words, the array is a DNA array in which a nucleic acid (sense nucleic acid or antisense nucleic acid) corresponding to at least one of the genes selected by the method described in Section 1. above or fragments thereof are aligned and immobilized. By the use of the array, there is exhibited an excellent property such that alterations in the expressions of the above-mentioned genes in a sample to be tested can be conveniently and precisely determined.

The phrase "immobilized at each of the defined region" used herein means that the region to which the nucleic acid (sense nucleic acid or antisense nucleic acid) corresponding to each of the genes or fragments thereof is immobilized is previously determined on the support. In other words, when the array as described above is used, there can be known which of the genes, nucleic acids or fragments thereof, the signals are ascribed, on the basis of the region of the detected signals.

From the viewpoint of more accurate classification of cancer, it is preferable that the DNA array described herein is one in which at least 5 kinds of genes selected from the marker genes listed in Table 1 above or their fragments, and/or at least 5 kinds of genes selected from the marker genes listed in Table 2 above or their fragments are immobilized, without being particularly limited thereto.

The support used in the DNA array described herein may be any of those which can be used for hybridization without particular limitation. Usually, a glass slide, a silicon chip, a nitrocellulose membrane, a nylon membrane or the like may be used. More preferably, a non-porous material with smooth surface may be used. For instance, glass such as a glass slide can be preferably used, without being particularly limited thereto. The surface of the support may be any of those in which a single-stranded DNA can be immobilized thereto via covalent bonding or non-covalent bonding. There can be preferably used a support having a hydrophilic or hydrophobic functional group on its surface, including, for instance, those having hydroxyl group, amino group, thiol group, aldehyde group, carboxyl group, acyl group, or the like, without being particularly limited thereto. Those functional groups may be present as those giving the surface characteristics of the support itself, or they may be introduced by subjecting the support to surface treatment. The surface-treated support as described above includes, for instance, those in which glass is treated with a commercially available silane coupling agent such as an aminoalkylsilane, glass treated with a polycation such as polylysine or polyethyleneimine, and the like. Some of the slide glass subjected to these treatments are commercially available.

In the DNA array described herein, either a single-stranded or double-stranded gene or its fragment may be immobilized thereto. For instance, there may be the DNA array in which the nucleic acids or fragments thereof in the form of a denatured double-strand are aligned and immobilized to a support, or the DNA array in which at least a part of the immobilized DNA is a single-stranded DNA. Alternatively, the array described herein may be a DNA array prepared by spotting double-stranded DNA under denaturation onto the same support in alignment.

The nucleic acid or its fragment to be immobilized to the support is not particularly limited, and any of polynucleotides or oligonucleotides may be used, as long as the level of mRNA transcribed from a marker gene can be determined. In addition, there are no particular limitation as to its preparation method, and there can be used any of those chemically synthesized, those isolated or purified from naturally-occurring nucleic acids, those enzymatically synthesized, or any of combination of these methods.

The nucleic acids or fragments thereof immobilized on a support, a double-stranded polynucleotide having 50 nucleotides or more in length, prepared by enzymatic amplification by the PCR (polymerase chain reaction), or derivatives thereof can be suitably used in the present invention. The derivatives are exemplified by those having modification such that they can be immobilized to the surface of a support. For instance, there is included a DNA into which a functional group such as amino group or thiol group is introduced at the 5'-end of DNA, without being particularly limited thereto. In the immobilization of the gene to a support, the above-mentioned double-stranded polynucleotide or a derivative thereof can be denatured, to give a single-stranded polynucleotide or a derivative thereof.

When the polynucleotide is immobilized to an array, the chain length of polynucleotide is not particularly limited. For instance, the polynucleotide having about 50 nucleotides in length to about 1 kilo nucleotides in length can be preferably used in the present invention. Those polynucleotides having a shorter or longer chain length than that defined above may also be used, as long as the polynucleotides are capable of specifically hybridizing to nucleic acids derived from the samples to be tested.

For instance, DNA resulting from amplification by, for instance, PCR with genomic DNA library or cDNA library as a template can be used. The array can be prepared by a known method, for instance, by immobilizing nucleic acids corresponding to the above-mentioned genes or fragments thereof on a support into which a functional group such as amino group is introduced. Alternatively, the above-described immobilization procedures are performed by using an DNA array producing apparatus such as DNA chip producing apparatus manufactured by GMS, whereby the array of the present invention to which nucleic acids corresponding to the genes are aligned and immobilized can be prepared.

Concretely, there are exemplified a DNA array in which at least 5 kinds of genes selected from the marker genes listed in Table 1 above or fragments thereof, and/or at least 5 kinds of genes selected from the marker genes listed in Table 2 above or fragments thereof are immobilized on the support.

Further, in the array of the present invention, the density at which the nucleic acids or fragments thereof are immobilized on the support is not particularly limited. For instance, the array may be one in which the DNAs are immobilized at a high density, and those in which the DNAs are immobilized at a density of 100 dots DNA/$cm^2$ or more can be preferably used. The high-density array as described above is commonly referred to as a "DNA microarray."

The high-density array is advantageous in the present invention, from the viewpoint of enabling high-sensitive and high-precision determination using a small amount of the sample.

The DNA array of the present invention can be prepared so that samples can be classified into plural numbers of groups. In this case, a group of genes used as indices for classification to each group are each adjoined on the support, and immobilized on the support separately from other group of genes. Therefore, the determination results can be analyzed quickly and conveniently.

The present invention will be further concretely described by means of Examples.

Example 1 RNA Extraction

Ten cases where there were histopathologically no lymph node metastasis (Sample Nos. D232, D242, D250, D272, D278, D288, D292, D294, D301 and D308) (Group A), and 10 cases where there were 5 or more lymph node metastases (Sample Nos. D230, D238, D244, D260, D285, D299, D300, D302, D304 and D305) (Group B) were selected from patients suffering from esophageal squamous cell carcinoma, to extract total RNA from a primary lesion of surgically excised specimens from each of the cases by using a reagent for RNA extraction ISOGEN (manufactured by Nippon Gene) in accordance with the method described in the instruction manual attached to the reagent.

Example 2 Northern Blot Analysis

Five micrograms of the total RNA obtained in Example 1 for each case was used to carry out Northern blot analysis using a nucleic acid encoding E2F-1 as a probe for Northern blot analysis.

Here, the above-mentioned probe for Northern blot analysis was prepared as follows. Concretely, RT-PCR (reverse-transcribed-PCR) was carried out by using a primer having the sequence of SEQ ID NO: 1 and a primer having the sequence of SEQ ID NO: 2 with 0.1 $\mu$g of human mRNA library (manufactured by ORIGENE) as a template. The resulting amplified product was subjected to 1% agarose gel electrophoresis. A portion corresponding to a band having a size of 520 bp was cut out from the gel after electrophoresis, and purified in accordance with a conventional method. About 100 ng of the purified DNA fragment was labeled with $^{32}$P-dCTP using Random Primer DNA Labeling Kit (manufactured by Boehringer Mannheim), to give a labeled E2F-1 probe for Northern blot analysis.

The Northern hybridization was carried out as follows. First, total RNA of each sample obtained in Example 1 was electrophoresed on formalin-denatured 1% agarose gel, 5 $\mu$g each per well. Thereafter, the gel after electrophoresis was blotted on nitrocellulose filter Nitro Plus™ (manufactured by Millipore Corporation), and RNA on the gel was transferred on the nitrocellulose filter.

Prehybridization was carried out by allowing the filter after the transfer to stand at 42°C for 2 hours in prehybridization buffer (50% formamide, 5 $\times$ SSC, 5 $\times$ Denhardt's solution, 0.1% SDS). Thereafter, the filter after the prehybridization was allowed to stand overnight at 42°C in hybridization buffer (40% formamide, 4 $\times$ SSC, 4 $\times$ Denhardt's solution, 0.08% SDS, 10% dextrin) added so that the above-mentioned labeled E2F-1 probe has a final concentration of 4 ng/ml. After the hybridization, the resulting filter was washed twice with a washing (0.1 $\times$ SSC, 0.1% SDS) at 65°C, 30 minutes. The filter after washing was exposed overnight to high-sensitive X-ray film (manufactured by Kodak). The signal intensity in the resulting autoradiogram was visually compared and studied.

As a result, of the 10 cases having 5 or more lymph node metastases, in the 4 cases (Sample Nos. D238, D260, D299 and D304), the expression of E2F-1 was high (Group B-H), and in the remaining 6 cases (Sample Nos. D230, D244, D285, D300, D302 and D305), the expression of E2F-1 was low (Group B-L). In addition, of the 10 cases having no lymph node metastasis, in one case (Sample No. D242), the expression of E2F-1 was high (Group A-H), and in the remaining 9 cases (Sample Nos. D232, D250, D272, D278, D288, D292, D294, D301 and D308), the expression of E2F-1 was low (Group A-L).

Example 3

Gene expression analysis was carried out as described below by using a total of 13 cases consisting of 4 cases having 5 or more lymph node metastases and high expression of E2F-1 (D260, D299, D304 and D238), 4 cases having 5 or more lymph node metastases and low expression of E2F-1 (D285, D300, D230 and D244), and 5 cases of having 1 to 3 lymph node metastases [D256 (with 1 lymph node metastasis), D258 (with 1 lymph node metastasis), D295 (with 1 lymph node metastasis), D296 (with 2 lymph node metastases), and D298 (with 3 lymph node metastases)] as samples and Group A-L which had no lymph node metastasis and low expression of E2F-1 as a control sample on Human Cancer CHIP (manufactured Takara Shuzo Co., Ltd.) and Human Apoptosis CHIP (manufactured by Takara Shuzo Co., Ltd.).

Total RNA was extracted for each of the above-mentioned cases in the same manner as in Example 1. cDNA was synthesized by using Time Saver™ cDNA Synthesis Kit (manufactured by Amersham Pharmacia) in accordance with the instruction manual attached to the kit, using T7-dT24 primer having the sequence of SEQ ID NO: 3 as a primer and 5 μg of the resulting total RNA as a template.

The resulting cDNA was each dissolved in 10 μl of TE buffer. cRNA was synthesized by using MEGAscript™ *in vitro* Transcription Kit for Large Scale Synthesis of RNAs (manufactured by Ambion) in accordance with the instruction manual attached to the kit with the resulting cDNA solution in an amount equivalent to 5 μl of as a template.

Reverse transcription reaction was carried out by using Cy3-dUTP as described below with 2 μg of each of the resulting cRNA as a template, to prepare DNA fluorescent-labeled with Cy3. Concretely, 20 μl of a reaction solution was prepared by mixing cRNA 2 μg, λpolyA$^+$ RNA-A (manufactured by Takara Shuzo Co., Ltd.) 7 ng, random primer hexadeoxyribonucleotide mix (manufactured by Takara Shuzo Co., Ltd.) 0.75 μg, and the solution was heated at 65°C for 10 minutes and then ice-cooled. RNase inhibitor 61.5 U, 10 × low dTNTP mix 4 μl, 1 mM Cy3-dUTP 4 μl, 5 × AMV reaction buffer 8 μl, AMV reverse transcriptase XL (manufactured by Life Science) 50 U were added to the solution after ice-cooling, and the reverse transcription reaction was carried out at 42°C for 1 hour with shading. Next, AMV reverse transcriptase XL 50 U was further added to the resulting reaction product, and the reverse transcription reaction was carried out at 42°C for 1 hour with shading. After the termination of the reaction, the resulting reaction product was purified by Centricep column (manufactured by Applied Biosystems), and the resulting purified product was subjected to ethanol precipitation. The recovered precipitate was dissolved in 5 μl of hybridization buffer (6 × SSC, 0.2% SDS, 5 × Denhardt's solution, heat denatured salmon sperm DNA 100 μg/ml, human CotI DNA 1.25 μg/μl, polydeoxyadenosine 0.8 μg/μl, yeast tRNA 1 μg/μl) to give Cy3-labeled sample DNA.

As to Group A-L, the RNA extraction, the cDNA synthesis and the cRNA synthesis were carried out in the same manner. The reverse transcription reaction using Cy5-dUTP was carried out with 2 μg of the resulting cDNA as a template, thereby fluorescence-labeling with Cy5, to give Cy5-labeled Group A-L DNA.

A mixture prepared by mixing an equal volume of each of Cy3-labeled sample DNA and Cy5-labeled Group A-L DNA was subjected to gene expression analysis on Human Cancer CHIP (manufactured Takara Shuzo Co., Ltd.) and Human Apoptosis CHIP (manufactured by Takara Shuzo Co., Ltd.). Here, the analysis on the CHIP was carried out as follows.

Ten microliters of prehybridization buffer (6 × SSC, 0.2% SDS, 5 × Denhardt's solution, heat denatured salmon sperm DNA 1 μg/μl) was dropped on a cover glass. Human Cancer CHIP (manufactured Takara Shuzo Co., Ltd.) or Human Apoptosis CHIP (manufactured by Takara Shuzo Co., Ltd.) was put on the dropped solution, and the surroundings were sealed with glue for paper. The resulting CHIP was kept at 65°C for 2 hours. Thereafter, the cover glass was taken off, and each of CHIP was washed with 2 × SSC, and then with 0.2 × SSC and air-dried. By the above treatments, the prehybridization was carried out.

Ten microliters of a solution prepared by mixing an equal volume of Cy3-labeled sample DNA and Cy5-labeled Group A-L DNA was heat-treated at 100°C for 2 minutes, and the heat-treated mixture was ice-cooled. The resulting solution was dropped on a cover glass. Human Cancer CHIP (manufactured Takara Shuzo Co., Ltd.) or Human Apoptosis CHIP (manufactured by Takara Shuzo Co., Ltd.) was put on the dropped solution, and the surroundings were sealed with glue for paper. The resulting CHIP was kept overnight at 65°C to carry out the hybridization. Thereafter, the cover glass was taken off, and each of CHIP was washed at 65°C in a solution of 2 × SSC, 0.2% SDS for 5 minutes and then twice at 55°C in a solution of 2 × SSC, 0.2% SDS for 30 minutes. Thereafter, each washed CHIP was rinsed with 0.05 × SSC for 5 minutes, and then air-dried.

Each of the resulting CHIP after hybridization was analyzed with a microscanner (manufactured by GMS) to determine fluorescent signals of each spot. The determined signal was analyzed with an expression data analysis software ImaGene (manufactured by BioDiscovery, Inc.).

As a result, the following genes:

- insulin-like growth factor binding protein 2 (IGFBP2) gene, (GenBank accession number: X16302);
- BIGH3 gene (GenBank accession number: M77349);
- insulin-like growth factor binding protein 6 (IGFBP6) gene, (GenBank accession number: M62402);
- gelatinase A (MMP-2) gene, (GenBank accession number: M55593);
- type II cytoskeletal 7 keratin (cytokeratin 7 (K7; CK 7)) gene, (GenBank accession number: M13955);
- desmoplakin I gene, (GenBank accession number: M77830);
- glutathione S-transferase A1 gene, (GenBank accession number: M16594);
- glutathione S-transferase Pi (GSTP1) gene, (GenBank accession number: U12472);
- collagenase-3 (MMP-13) gene, (GenBank accession number: X75308);
- type II cytoskeletal 5 keratin (cytokeratin 5 (K5; CK 5)) gene, (GenBank accession number: M21389);
- P-cadherin gene, (GenBank accession number: X63629);
- type I cytoskeletal 14 keratin (cytokeratin 14 (K14; CK 14)) gene, (GenBank accession number: J00124); and

- type II cytoskeletal 6 keratin (cytokeratin 6B (CK 6B)) gene, (GenBank accession number: L42610)

showed reduction in the expression levels in the samples having metastasis.

Also, the following genes:

- matrilysin (MMP-7) gene, (GenBank accession number: X07819);
- forkhead-like 7 gene, (GenBank accession number: AF048693);
- connective tissue growth factor (CTGF) gene, (GenBank accession number: M92934);
- growth hormone-dependent insulin-like growth factor-binding protein gene, (GenBank accession number: M35878); and
- Rho8 protein gene, (GenBank accession number: X95282) showed reduction in the expression levels in some of the samples having metastasis.

On the other hand, the following genes:

- RBA/p48 gene (GenBank accession number: X74262);
- cell division control protein 2 homolog (EC 2.7.1.-) (cdc2) gene, (GenBank accession number: X05360);
- replication factor C 38-kDa subunit (RFC38) gene, (GenBank accession number: L07541);
- apopain precursor gene, (GenBank accession number: U13737);
- xeroderma pigmentosum group C repair complementing protein p58/HHR23B gene, (GenBank accession number: D21090);
- cyclin G2 gene, (GenBank accession number: U47414);
- cyclin A gene, (GenBank accession number: X51688);
- apoptosis-related protein TFAR15 gene, (GenBank accession number: AF022385);
- TRKB tyrosine kinase receptor gene, (GenBank accession number: U12140); and
- gene for signal transducer and activator of transcription 1-alpha/beta (STAT1), (GenBank accession number: M97935)

showed no difference in the expression levels in the samples having 1 to 3 lymph node metastases with the expression level in the control sample, but had an increase in the expression level in the sample having 5 lymph node metastases.

In addition, the following genes:

- K-ras oncogene, (GenBank accession number: M54968);
- retinoblastoma susceptibility protein (RB1) gene, (GenBank accession number: L41870);
- gene for BCL2/adenovirus E1B 19kD-interacting protein 1 (BNIP1) mRNA, complete cds, (GenBank accession number: AF083957); and
- inhibitor of apoptosis protein 1 (HIAP-1) gene, (GenBank accession number: U45878)

showed increase in the expression levels in some of the samples having 5 or more lymph node metastases.

Increase in the expression levels of the following genes:

- cyclin C G1/S-specific gene (GenBank accession number: M74091);
- BRCA1-associated ring domain protein gene (GenBank accession number: U76638);
- APC gene (GenBank accession number: M73548);
- integrin alpha-E precursor (ITGAE) gene (GenBank accession number: L25851);
- ezrin (cytovillin 2) gene (GenBank accession number: X51521);
- recA-like protein HsRad51 gene (GenBank accession number: L07493);
- vascular endothelial growth factor C precursor (VEGF-C) gene (GenBank accession number: U43142); and
- cyclin E gene (GenBank accession number: M73812)

was found in samples having 5 or more metastases, but the samples in which increase in these 8 kinds of gene expressions was seen were all low in E2F-1 expression.

Also, increase in the expression levels of

- gene for CDK6 inhibitor 2c (p18) mRNA, complete cds (GenBank accession number: U17074); and
- PCNA gene (GenBank accession number: M15796) was found in samples having 5 or more metastases, but the samples in which increase in these 2 kinds of gene expressions was seen were all high in E2F-1 expression.

Therefore, these 10 kinds of genes are considered as genes in which the expression level increases or decreases according to the level of E2F-1 expression.

It is clarified from the above results that

- insulin-like growth factor binding protein 2 (IGFBP2) gene, (GenBank accession number: X16302);
- BIGH3 gene (GenBank accession number: M77349);
- insulin-like growth factor binding protein 6 (IGFBP6) gene, (GenBank accession number: M62402);
- gelatinase A (MMP-2) gene, (GenBank accession number: M55593);
- type II cytoskeletal 7 keratin (cytokeratin 7 (K7; CK 7)) gene, (GenBank accession number: M13955);
- desmoplakin I gene, (GenBank accession number: M77830);
- glutathione S-transferase A1 gene, (GenBank accession number: M16594);
- glutathione S-transferase Pi (GSTP1) gene, (GenBank accession number: U12472);
- collagenase-3 (MMP-13) gene, (GenBank accession number: X75308);
- type II cytoskeletal 5 keratin (cytokeratin 5 (K5; CK 5)) gene, (GenBank accession number: M21389);
- P-cadherin gene, (GenBank accession number: X63629);
- type I cytoskeletal 14 keratin (cytokeratin 14 (K14; CK 14)) gene, (GenBank accession number: J00124);
- type II cytoskeletal 6 keratin (cytokeratin 6B (CK 6B)) gene, (GenBank accession number: L42610);
- matrilysin (MMP-7) gene, (GenBank accession number: X07819);
- forkhead-like 7 gene, (GenBank accession number: AF048693);
- connective tissue growth factor (CTGF) gene, (GenBank accession number: M92934);
- growth hormone-dependent insulin-like growth factor-binding protein gene, (GenBank accession number: M35878); and
- Rho8 protein gene, (GenBank accession number: X95282), especially preferably
- insulin-like growth factor binding protein 2 (IGFBP2) gene, (GenBank accession number: X16302);
- BIGH3 gene (GenBank accession number: M77349);
- insulin-like growth factor binding protein 6 (IGFBP6) gene, (GenBank accession number: M62402);
- gelatinase A (MMP-2) gene, (GenBank accession number: M55593);
- type II cytoskeletal 7 keratin (cytokeratin 7 (K7; CK 7)) gene, (GenBank accession number: M13955);
- desmoplakin I gene, (GenBank accession number: M77830);
- glutathione S-transferase A1 gene, (GenBank accession number: M16594);
- glutathione S-transferase Pi (GSTP1) gene, (GenBank accession number: U12472);
- collagenase-3 (MMP-13) gene, (GenBank accession number: X75308);
- type II cytoskeletal 5 keratin (cytokeratin 5 (K5; CK 5)) gene, (GenBank accession number: M21389);
- P-cadherin gene, (GenBank accession number: X63629);
- type I cytoskeletal 14 keratin (cytokeratin 14 (K14; CK 14)) gene, (GenBank accession number: J00124); and
- type II cytoskeletal 6 keratin (cytokeratin 6B (CK 6B)) gene, (GenBank accession number: L42610)

are indices for lymph node metastasis.

Further, it is clarified that

- RBA/p48 gene (GenBank accession number: X74262);
- cell division control protein 2 homolog (EC 2.7.1.-)(cdc2) gene, (GenBank accession number: X05360);
- replication factor C 38-kDa subunit (RFC38) gene, (GenBank accession number: L07541);
- apopain precursor gene, (GenBank accession number: U13737);
- xeroderma pigmentosum group C repair complementing protein p58/HHR23B gene, (GenBank accession number: D21090);
- cyclin G2 gene, (GenBank accession number: U47414);
- cyclin A gene, (GenBank accession number: X51688);
- apoptosis-related protein TFAR15 gene, (GenBank accession number: AF022385);
- TRKB tyrosine kinase receptor gene, (GenBank accession number: U12140);
- gene for signal transducer and activator of transcription 1-alpha/beta (STAT1), (GenBank accession number: M97935);
- K-ras oncogene, (GenBank accession number: M54968);
- retinoblastoma susceptibility protein (RB1) gene, (GenBank accession number: L41870); and
- gene for BCL2/adenovirus E1B 19kD-interacting protein 1 (BNIP1) mRNA, complete cds, (GenBank accession number: AF083957), especially preferably
- RBA/p48 gene (GenBank accession number: X74262);
- cell division control protein 2 homolog (EC 2.7.1.-) (cdc2) gene, (GenBank accession number: X05360);

- replication factor C 38-kDa subunit (RFC38) gene, (GenBank accession number: L07541);
- apopain precursor gene, (GenBank accession number: U13737);
- xeroderma pigmentosum group C repair complementing protein p58/HHR23B gene, (GenBank accession number: D21090);
- cyclin G2 gene, (GenBank accession number: U47414);
- cyclin A gene, (GenBank accession number: X51688);
- apoptosis-related protein TFAR15 gene, (GenBank accession number: AF022385);
- TRKB tyrosine kinase receptor gene, (GenBank accession number: U12140); and
- gene for signal transducer and activator of transcription 1-alpha/beta (STAT1), (GenBank accession number: M97935)

are indices for high lymph node metastasis.

Example 4

From the genes clarified to be indices of lymph nodes in Example 3, 5 kinds of genes:

- type I cytoskeletal 14 keratin (cytokeratin 14 (K14; CK 14)) gene, (GenBank accession number: J00124);
- type II cytoskeletal 6 keratin (cytokeratin 6B (CK 6B)) gene, (GenBank accession number: L42610);
- type II cytoskeletal 5 keratin (cytokeratin 5 (K5; CK 5)) gene, (GenBank accession number: M21389);
- gelatinase A (MMP-2) gene, (GenBank accession number: M55593); and
- type II cytoskeletal 7 keratin (cytokeratin 7 (K7; CK 7)) gene, (GenBank accession number: M13955)

were selected, and a DNA microarray to which cDNA corresponding to each of these genes was immobilized was prepared as follows.

cDNA fragment was amplified by RT-PCR method for each of the genes with RNA of Group A-L prepared in Example 3 as a template. There were used a primer pair consisting of primers each having the sequence of SEQ ID NO: 4 or 5 in the cDNA amplification of the type I cytoskeletal 14 keratin (cytokeratin 14 (K14; CK 14)), (GenBank accession number: J00124); a primer pair consisting of primers having the sequences of SEQ ID NOs: 6 and 7 in the cDNA amplification of the type II cytoskeletal 6 keratin (cytokeratin 6B (CK 6B)), (GenBank accession number: L42610); a primer pair consisting of primers having the sequences of SEQ ID NOs: 8 and 9 in the cDNA amplification of the type II cytoskeletal 5 keratin (cytokeratin 5 (K5; CK 5)), (GenBank accession number: M21389); a primer pair consisting of primers having the sequences of SEQ ID NOs: 10 and 11 in the cDNA amplification of the gelatinase A (MMP-2), (GenBank accession number: M55593); and a primer pair consisting of primers having the sequences of SEQ ID NOs: 12 and 13 in the cDNA amplification of the type II cytoskeletal 7 keratin (cytokeratin 7 (K7; CK 7)), (GenBank accession number: M13955). The nucleotide sequence analysis of the amplified cDNA was performed, whereby confirming that the fragment is the desired one. In addition, the cDNA fragment confirmed as the desired fragment was recovered by ethanol precipitation method and dissolved in 10 mM carbonate buffer (pH 9.5) so as to have a concentration of 1 μM.

Each of cDNA of E1F-2 gene, cDNA of β-actin gene as a housekeeping gene, and a plasmid pUC18 as a negative control was prepared in the same manner.

Each of these DNA was spotted on amino group-coated slide glass (manufactured by Sigma) by using a DNA chip-producing apparatus (manufactured by GMS), and immobilized on the slide glass by UV irradiation. The resulting slide was washed with 0.2% SDS solution and then with distilled water, and dried, to give a DNA array.

Example 5

The following kit was constructed.

(1) Kit 1

1) Primer and primer for determining each of mRNA amount of the following 5 kinds of genes:

- type I cytoskeletal 14 keratin (cytokeratin 14 (K14; CK 14)) gene, (GenBank accession number: J00124);
- type II cytoskeletal 6 keratin (cytokeratin 6B (CK 6B)) gene, (GenBank accession number: L42610);
- type II cytoskeletal 5 keratin (cytokeratin 5 (K5; CK 5)) gene, (GenBank accession number: M21389);
- gelatinase A (MMP-2) gene, (GenBank accession number: M55593); and

- type II cytoskeletal 7 keratin (cytokeratin 7 (K7; CK 7)) gene, (GenBank accession number: M13955),

among the genes clarified to be indices for lymph node metastasis in Example 3;

| | | |
|---|---|---|
| 2) | AMV Reverse Transcriptase XL (5 U/μl) | 50 μl |
| 3) | RNase Inhibitor (40 U/μl) | 25 μl |
| 4) | Random 9mers (50 μM) | 50 μl |
| 5) | oligo dT (2.5 μM) | 50 μl |
| 6) | TaKaRa Taq (5 U/μl) | 25 μl |
| 7) | 10 × RNA PCR buffer (100 mM Tris-HCl, 500 mM KCl, pH 8.3) | 1 ml |
| 8) | dNTP mixture (10 mM each) | 150 μl |
| 9) | $MgCl_2$ (25 mM) | 1 ml |

(2) Kit 2

1) Antibody (labeled antibody) specifically binding to a polypeptide encoded by each of the following 5 genes:

- type I cytoskeletal 14 keratin (cytokeratin 14 (K14; CK 14)) gene, (GenBank accession number: J00124);
- type II cytoskeletal 6 keratin (cytokeratin 6B (CK 6B)) gene, (GenBank accession number: L42610);
- type II cytoskeletal 5 keratin (cytokeratin 5 (K5; CK 5)) gene, (GenBank accession number: M21389);
- gelatinase A (MMP-2) gene, (GenBank accession number: M55593); and
- type II cytoskeletal 7 keratin (cytokeratin 7 (K7; CK 7)) gene, (GenBank accession number: M13955)

among the genes clarified to be indices for lymph node metastasis in Example 3.

## SEQUENCE FREE TEXT

SEQ ID NO: 1 is a sequence for PCR primer to amplify a portion of E2F-1 gene.
SEQ ID NO: 2 is a sequence for PCR primer to amplify a portion of E2F-1 gene.
SEQ ID NO: 3 is a sequence for primer for reverse transcription.
SEQ ID NO: 4 is a sequence for PCR primer for to amplify a portion of type I cytoskeletal 14 keratin gene.
SEQ ID NO: 5 is a sequence for PCR primer for to amplify a portion of type I cytoskeletal 14 keratin gene
SEQ ID NO: 6 is a sequence for PCR primer for to amplify a portion of type II cytoskeletal 6 keratin gene.
SEQ ID NO: 7 is a sequence for PCR primer for to amplify a portion of type II cytoskeletal 6 keratin gene.
SEQ ID NO: 8 is a sequence for PCR primer for to amplify a portion of type II cytoskeletal 5 keratin gene.
SEQ ID NO: 9 is a sequence for PCR primer for to amplify a portion of type II cytoskeletal 5 keratin gene.
SEQ ID NO: 10 is a sequence for PCR primer for to amplify a portion of gelatinase A gene.
SEQ ID NO: 11 is a sequence for PCR primer for to amplify a portion of gelatinase A gene.
SEQ ID NO: 12 is a sequence for PCR primer for to amplify a portion of type II cytoskeletal 17 keratin gene.
SEQ ID NO: 13 is a sequence for PCR primer for to amplify a portion of type II cytoskeletal 17 keratin gene.

## INDUSTRIAL APPLICABILITY

According to the method for selecting a gene used as an index of cancer classification of the present invention, there can be conveniently and quickly selected a gene which is capable of performing classification in various cancers by their progressive stage, differentiation degree, type or the like, and can be provided information for the diagnosis or treatment of cancer. In addition, according to the method for classifying cancer of the present invention, the classification of cancer, for instance, progressive stage, differentiation degree, type and the like, can be carried out conveniently and quickly, whereby the selection of an appropriate method of treatment for individual cases can be made on the basis of the classification results. Further, according to the method for detecting cancer of the present invention, various progressive stages of cancer, various differentiation degrees of cancer, and various types of cancer can be detected conveniently and quickly, whereby the selection of an appropriate method of treatment can be made for individual cases. Therefore, the present invention is useful in the diagnosis, the treatment or the like of cancer.

## Claims

**1.** A method for selecting a gene used as an index of cancer classification, comprising the following steps of:

(1) determining expression levels in cancer samples to be tested for at least one of genes each of which expression is altered specifically during cell proliferation, and then comparing the determined expression levels with an expression level of the genes in a control sample, thereby evaluating alterations in expression levels of the genes, wherein the control sample is a normal tissue, or a cancer sample with low malignancy;
(2) classifying the cancer samples to be tested into plural numbers of types, based on alterations in expression levels of the genes evaluated in the above step (1) and pathological findings for the cancer samples to be tested; and
(3) examining alterations in expressions for plural numbers of genes in each of the cancer samples to be tested classified in the above step (2), to select a gene, wherein expression of said gene is altered independently to genes each of which expression is altered specifically during cell proliferation and expression level of said gene is specifically altered depending on every type of cancer samples to be tested,

wherein said genes each of which expression is altered specifically during cell proliferation are selected from the group consisting of CDC6 gene and E2F family genes.

**2.** The method according to claim 1, wherein in the step (1), an expression level of E2F-1 gene is determined.

**3.** The method according to any one of claims 1 or 2, wherein the expression levels of genes are determined on the basis of levels of mRNAs transcribed from the genes.

**4.** The method according to claim 3, wherein the levels of mRNAs are determined by a hybridization method or a nucleic acid amplification method.

**5.** The method according to claim 4, wherein the levels of mRNAs are determined by a DNA array-based hybridization method.

**6.** The method according to any one of claims 1 to 5, wherein in the step (2), the cancer samples to be tested are classified on the basis of pathological findings selected from the group consisting of cellular morphology, states of infiltration to peripheral tissues, sensitivity against drugs, and states of metastasis into lymph node.

**7.** A method for classifying a cancer, **characterized in that** cancer is classified with expression levels of genes in a sample to be tested, the method comprising the following steps:

(a) determining expression levels of at least one of genes used as indices of cancer classification, wherein the genes are selected by the selection method of any one of claims 1 to 6, in the sample to be tested, and
(b) comparing the gene expression levels determined in the step (a) with expression levels of the same genes in a control sample, thereby classifying cancer for the sample to be tested.

**8.** The method according to claim 7, wherein in the step (a), expression levels of at least 5 kinds of genes are determined.

**9.** The method according to claim 7 or 8, wherein the expression levels of genes are determined on the basis of levels of mRNAs transcribed from the gene, or levels of polypeptides translated from the genes.

**10.** The method according to claim 9, wherein the levels of mRNAs are determined by a hybridization method or a nucleic acid amplification method.

**11.** The method according to claim 10, wherein the levels of mRNAs are determined by a DNA microarray-based hybridization method.

**12.** The method according to claim 9, wherein the levels of polypeptides are determined by using antibodies capable of binding specifically to the polypeptides or fragments thereof.

**13.** The method according to any one of claims 8 to 12, wherein expression levels of at least 5 kinds of genes selected from the following Group I and/or expression levels of at least 5 kinds of genes selected from the following Group II are determined:

Group I:

- insulin-like growth factor binding protein 2 (IGFBP2) gene, (GenBank accession number: X16302); BIGH3 gene (GenBank accession number: M77349);
- insulin-like growth factor binding protein 6 (IGFBP6) gene, (GenBank accession number: M62402);
- gelatinase A (MMP-2) gene, (GenBank accession number: M55593);
- type II cytoskeletal 7 keratin (cytokeratin 7 (K7; CK 7)) gene, (GenBank accession number: M13955);
- -desmoplakin I gene, (GenBank accession number: M77830);
- glutathione S-transferase A1 gene, (GenBank accession number: M16594);
- glutathione S-transferase Pi (GSTP1) gene, (GenBank accession number: U12472);

- collagenase-3 (MMP-13) gene, (GenBank accession number: X75308);
- type II cytoskeletal 5 keratin (cytokeratin 5 (K5; CK 5)) gene, (GenBank accession number: M21389);
- P-cadherin gene, (GenBank accession number: X63629);
- type I cytoskeletal 14 keratin (cytokeratin 14 (K14; CK 14)) gene, (GenBank accession number: J00124);
- type II cytoskeletal 6 keratin (cytokeratin 6B (CK 6B)) gene, (GenBank accession number: L42610);
- matrilysin (MMP-7) gene, (GenBank accession number: X07819);
- forkhead-like 7 gene, (GenBank accession number: AF048693);
- connective tissue growth factor (CTGF) gene, (GenBank accession number: M92934);
- growth hormone-dependent insulin-like growth factor-binding protein gene, (GenBank accession number: M35878); and
- Rho8 protein gene, (GenBank accession number: X95282);

Group II:

- RBA/p48 gene (GenBank accession number: X74262);
- cell division control protein 2 homolog (EC 2.7.1.-) (cdc2) gene, (GenBank accession number: X05360);
- replication factor C 38-kDa subunit (RFC38) gene, (GenBank accession number: L07541);
- apopain precursor gene, (GenBank accession number: U13737);
- xeroderma pigmentosum group C repair complementing protein p58/HHR23B gene, (GenBank accession number: D21090);
- cyclin G2 gene, (GenBank accession number: U47414);
- cyclin A gene, (GenBank accession number: X51688);
- apoptosis-related protein TFAR15 gene, (GenBank accession number: AF022385);
- TRKB tyrosine kinase receptor gene, (GenBank accession number: U12140);
- gene for signal transducer and activator of transcription 1-alpha/beta (STAT1), (GenBank accession number: M97935)
- K-ras oncogene, (GenBank accession number: M54968);
- retinoblastoma susceptibility protein (RB1) gene, (GenBank accession number: L41870);
- gene for BCL2/adenovirus E1B 19kD-interacting protein 1 (BNIP1) mRNA, complete cds, (GenBank accession number: AF083957); and
- inhibitor of apoptosis protein 1 (HIAP-1) gene, (GenBank accession number: U45878).

**14.** The method according to any one of claims 7 to 13, wherein a sample to be tested is classified on the basis of risk for metastasis into lymph node.

**15.** A method for detecting cancer, comprising the following steps:

(1) determining expression levels of at least one of genes used as indices of cancer classification in a sample to be tested, wherein the genes are selected by the selection method of any one of claims 1 to 6, and
(2) comparing the expression levels of genes in the sample to be tested determined in the step (1) with expression levels of the genes in a control sample, thereby detecting cancer,

wherein expressions of nucleic acids corresponding to at least one of genes or expressions of polypeptides encoded by at least one of the genes used as indices of cancer classification are altered compared with a control sample is an index of the presence of cancer cells in the sample to be tested.

**16.** The method according to claim 15, wherein in the step (1), expression levels of at least 5 kinds of genes are determined.

**17.** The method according to claim 15 or 16, wherein the expression levels of genes are determined on the basis of levels of mRNAs transcribed from the genes, or levels of polypeptides translated from the genes.

**18.** The method according to claim 17, wherein the levels of mRNAs are determined by a hybridization method or a nucleic acid amplification method.

**19.** The method according to claim 18, wherein the levels of mRNAs are determined by a DNA microarray-based hybridization method.

**20.** The method according to claim 17, wherein the levels of polypeptides are determined by using antibodies capable of binding specifically to the polypeptides or fragments thereof.

**21.** The method according to any one of claims 15 to 20 wherein expression levels of at least 5 kinds of genes selected from genes of Group I of claim 13, and/or expression levels of at least 5 kinds of genes selected from genes of Group II of claim 13 are determined.

**Patentansprüche**

**1.** Verfahren zum Auswählen eines Gens, das als Anzeige zur Einstufung von Krebs verwendet wird, umfassend die folgenden Schritte:

(1) Bestimmen der Expressionsspiegel in zu testenden Krebsproben von mindestens einem von Genen, wobei jede Expression während der Zellproliferation spezifisch verändert wird, und danach Vergleichen der bestimmten Expressionsspiegel mit einem Expressionsspiegel der Gene in einer Kontrollprobe, wodurch Veränderungen in den Expressionsspiegeln der Gene bewertet werden, wobei die Kontrollprobe ein normales Gewebe oder eine Krebsprobe mit geringer Malignität ist;
(2) Einstufen der zu testenden Krebsproben in eine Vielzahl von Typen basierend auf Veränderungen in den Expressionsspiegeln der Gene, die in vorstehendem Schritt (1) bewertet wurden, und auf pathologischen Ergebnissen bei den zu testenden Krebsproben; und
(3) Untersuchen von Veränderungen in der Expression für eine Vielzahl von Genen in jeder der zu testenden Krebsproben, die in vorstehendem Schritt (2) eingestuft wurden, um ein Gen auszuwählen, wobei die Expression des Gens unabhängig von Genen verändert wird, wobei jede Expression spezifisch während der Zellproliferation verändert wird und

wobei der Expressionsspiegel des Gens abhängig von jeder Art zu testender Krebsproben spezifisch verändert wird, wobei die Gene, bei denen jede Expression spezifisch während der Zellproliferation verändert wird, ausgewählt sind aus der Gruppe bestehend aus CDC6-Gen und Genen der E2F-Familie.

**2.** Verfahren nach Anspruch 1, wobei in Schritt (1) ein Expressionsspiegel des E2F-1-Gens bestimmt wird.

**3.** Verfahren nach einem der Ansprüche 1 oder 2, wobei die Expressionsspiegel von Genen auf der Basis von von den Genen transkribierten mRNA-Spiegeln bestimmt werden.

**4.** Verfahren nach Anspruch 3, wobei die mRNA-Spiegel durch ein Hybridisierungsverfahren oder ein Nucleinsäureamplifikationsverfahren bestimmt werden.

**5.** Verfahren nach Anspruch 4, wobei die mRNA-Spiegel durch ein auf DNA-Array basierendes Hybridisierungsverfahren bestimmt werden.

**6.** Verfahren nach einem der Ansprüche 1 bis 5, wobei in Schritt (2) die zu testenden Krebsproben auf der Basis pathologischer Ergebnisse eingestuft werden, die ausgewählt sind aus der Gruppe bestehend aus Zellmorphologie, Infiltrationsstatus peripherer Gewebe, Empfindlichkeit gegen Wirkstoffe und Status von Metastasen in Lymphknoten.

**7.** Verfahren zum Einstufen eines Krebses, **dadurch gekennzeichnet, dass** Krebs mit Expressionsspiegeln von Genen in einer zu testenden Probe eingestuft wird, wobei das Verfahren die folgenden Schritte umfasst:

(a) Bestimmen der Expressionsspiegel in der zu testenden Probe von mindestens einem von Genen, die als Anzeige der Krebseinstufung verwendet werden, wobei die Gene mittels des Auswahlverfahrens nach einem der Ansprüche 1 bis 6 ausgewählt werden, und
(b) Vergleichen der in Schritt (a) bestimmten Genexpressionsspiegel mit Expressionsspiegeln der gleichen Gene in einer Kontrollprobe, wodurch der Krebs für die zu testende Probe eingestuft wird.

**8.** Verfahren nach Anspruch 7, wobei in Schritt (a) die Expressionsspiegel von mindestens 5 Arten von Genen bestimmt werden.

**9.** Verfahren nach Anspruch 7 oder 8, wobei die Expressionsspiegel von Genen auf der Basis von von dem Gen transkribierten mRNA-Spiegeln oder von Spiegeln von von diesen Genen translatierten Polypeptiden bestimmt werden.

**10.** Verfahren nach Anspruch 9, wobei die mRNA-Spiegel durch ein Hybridisierungsverfahren oder ein Nucleinsäureamplifikationsverfahren bestimmt werden.

**11.** Verfahren nach Anspruch 10, wobei die mRNA-Spiegel durch ein auf DNA-Mikroarray basierendes Hybridisierungsverfahren bestimmt werden.

**12.** Verfahren nach Anspruch 9, wobei die Polypeptidspiegel unter Verwendung von Antikörpern bestimmt werden, die spezifisch an die Polypeptide oder Fragmente davon binden können.

**13.** Verfahren nach einem der Ansprüche 8 bis 12, wobei die Expressionsspiegel von mindestens 5 Arten von Genen ausgewählt aus der folgenden Gruppe I und/oder die Expressionsspiegel von mindestens 5 Arten von Genen ausgewählt aus der folgenden Gruppe II bestimmt werden:

Gruppe I:

- Insulinähnlicher Wachstumsfaktor-Bindeprotein 2(IGFBP2)-Gen, (Genbank-Zugangsnummer: X16302);
- BIGH3-Gen (Genbank-Zugangsnummer: M77349);
- Insulinähnlicher Wachstumsfaktor-Bindeprotein 6(IGFBP6)-Gen, (Genbank-Zugangsnummer: M62402);
- Gelatinase A-(MMP-2)-Gen, (Genbank-Zugangsnummer: M55593);
- Typ II-Cytoskelett 7-Keratin(Cytokeratin 7 (K7; CK 7))-Gen, (Genbank-Zugangsnummer: M13955);
- Desmoplakin I-Gen, (Genbank-Zugangsnummer: M77830);
- Glutathion-S-Transferase A1-Gen, (Genbank-Zugangsnummer: M 16594);
- Glutathion-S-Transferase Pi(GSTP1)-Gen, (Genbank-Zugangsnummer: U 12472);
- Collagenase-3(MMP-13)-Gen, (Genbank-Zugangsnummer: X75308);
- Typ II-Cytoskelett 5-Keratin(Cytokeratin 5 (K5, CK 5))-Gen, (Genbank-Zugangsnummer: M21389);
- P-Cadherin-Gen, (Genbank-Zugangsnummer: X63629);
- Typ I-Cytoskelett 14-Keratin(Cytokeratin 14 (K14, CK 14))-Gen, (Genbank-Zugangsnummer: J00124);
- Typ II-Cytoskelett 6-Keratin(Cytokeratin 6B (CK 6B))-Gen, (Genbank-Zugangsnummer: L4261 0);
- Matrilysin(MMP-7)-Gen, (Genbank-Zugangsnummer: X07819);
- Forkhead-like 7-Gen, (Genbank-Zugangsnummer: AF048693);
- Bindegewebewachstumsfaktor (CTGF)-Gen, (Genbank-Zugangsnummer: M92934);
- Wachstumsfaktor-abhängiges Insulin-ähnlicher Wachstumsfaktor-Bindeprotein-Gen, (Genbank-Zugangsnummer: M35878); und
- Rho8 Protein-Gen, (Genbank-Zugangsnummer: X95282);

Gruppe II:

- RBA/p48-Gen, (Genbank-Zugangsnummer: X74262);
- Zellteilungskontrollprotein 2-Homolog(EC 2.7.1.-) (cdc2)-Gen, (Genbank-Zugangsnummer: X05360);
- Replikationsfaktor C 38-kDa-Untereinheit(RFC38)-Gen, (Genbank-Zugangsnummer: L07541);
- Apopainvorläufergen, (Genbank-Zugangsnummer: U13737);
- Xeroderma pigmentosum-Gruppe C-Reparaturkomplementierungsprotein p58/HHR23B-Gen, (Genbank-Zugangsnummer: D21090);
- Cyclin G2-Gen, (Genbank-Zugangsnummer: U47414);
- Cyclin A-Gen, (Genbank-Zugangsnummer: X51688);
- Apoptose-bezogenes Protein TFAR15-Gen, (Genbank-Zugangsnummer: AF022385);
- TRKB-Tyrosinkinaserezeptor-Gen, (Genbank-Zugangsnummer: U12140);
- Gen für Signalüberträger und Transkriptionsaktivator 1-alpha/beta (STAT1), (Genbank-Zugangsnummer: M97935);
- K-ras-Onkogen, (Genbank-Zugangsnummer: M54968);
- Retinoblastom-Prädispositionsprotein(RB1)-Gen, (Genbank-Zugangsnummer: L41870);
- Gen für das BCL2/Adenovirus E1B-19kD-interagierende Protein 1 (BNIP1)-mRNA, vollständige cds, (Genbank-Zugangsnummer: AF083957); und
- Gen für den Inhibitor des Apoptoseproteins 1 (HIAP-1), (Genbank-Zugangsnummer: U45878).

**14.** Verfahren nach einem der Ansprüche 7 bis 13, wobei eine zu testende Probe auf der Basis des Risikos für Metastasen in Lymphknoten eingestuft wird.

**15.** Verfahren zum Nachweisen von Krebs umfassend die folgenden Schritte:

(1) Bestimmen der Expressionsspiegel in der zu testenden Probe von mindestens einem von Genen, die als Anzeige der Krebseinstufung verwendet werden, wobei die Gene mittels des Auswahlverfahrens nach einem der Ansprüche 1 bis 6 ausgewählt werden, und
(2) Vergleichen der Expressionsspiegel der Gene in der zu testenden Probe, die in Schritt (1) bestimmt wurden, mit Expressionsspiegeln der Gene in einer Kontrollprobe, wodurch Krebs nachgewiesen wird,

wobei die Expressionen von Nucleinsäuren, die mindestens einem der Gene entsprechen, oder die Expressionen von Polypeptiden, die durch mindestens eines der als Anzeige der Krebseinstufung verwendeten Gene codiert werden, im Vergleich mit einer Kontrollprobe verändert sind, was eine Anzeige für die Anwesenheit von Krebszellen in der zu testenden Probe ist.

**16.** Verfahren nach Anspruch 15, wobei in Schritt (1) die Expressionsspiegel von mindestens 5 Arten von Genen bestimmt werden.

**17.** Verfahren nach Anspruch 15 oder 16, wobei die Expressionsspiegel von Genen auf der Basis von von den Genen transkribierten mRNA-Spiegeln oder von Spiegeln von von diesen Genen translatierten Polypeptiden bestimmt werden.

**18.** Verfahren nach Anspruch 17, wobei die mRNA-Spiegel durch ein Hybridisierungsverfahren oder ein Nucleinsäureamplifikationsverfahren bestimmt werden.

**19.** Verfahren nach Anspruch 18, wobei die mRNA-Spiegel durch ein auf DNA-Mikroarray basierendes Hybridisierungsverfahren bestimmt werden.

**20.** Verfahren nach Anspruch 17, wobei die Polypeptidspiegel unter Verwendung von Antikörpern bestimmt werden, die spezifisch an die Polypeptide oder Fragmente davon binden können.

**21.** Verfahren nach einem der Ansprüche 15 bis 20, wobei die Expressionsspiegel von mindestens 5 Arten von Genen ausgewählt aus Genen der Gruppe I nach Anspruch 13 und/oder Expressionsspiegel von mindestens 5 Arten von Genen ausgewählt aus den Genen der Gruppe II nach Anspruch 13 bestimmt werden.

## Revendications

**1.** Procédé de sélection d’un gène utilisé comme indice de classification de cancers, comprenant les étapes suivantes consistant à :

(1) déterminer les taux d’expression dans des échantillons cancéreux à tester pour au moins un des gènes dont l’expression de chacun est modifiée spécifiquement au cours de la prolifération cellulaire, et ensuite comparer les taux d’expression déterminés avec un taux d’expression des gènes dans un échantillon contrôle, évaluant de cette manière des modifications des taux d’expression des gènes, où l’échantillon contrôle est un tissu normal ou un échantillon cancéreux de faible malignité ;
(2) classer les échantillons cancéreux à tester en plusieurs nombres de types, en se basant sur les modifications des taux d’expression des gènes évalués dans l’étape (1) ci-dessus et des découvertes pathologiques pour les échantillons cancéreux à tester ; et
(3) examiner les modifications des expressions pour plusieurs nombres de gènes dans chacun des échantillons cancéreux à tester classés dans l’étape (2) ci-dessus, pour sélectionner un gène, où l’expression dudit gène est modifiée indépendamment des gènes dont l’expression de chacun est modifiée spécifiquement au cours de la prolifération cellulaire et le taux d’expression dudit gène est spécifiquement modifié selon chaque type d’échantillons cancéreux à tester,

où lesdits gènes dont l’expression de chacun est modifiée spécifiquement au cours de la prolifération cellulaire sont choisis dans le groupe constitué du gène CDC6 et des gènes de la famille E2F.

**2.** Procédé selon la revendication 1, dans lequel dans l'étape (1), un taux d'expression du gène E2F-1 est déterminé.

**3.** Procédé selon l'une quelconque des revendications 1 ou 2, dans lequel les taux d'expression des gènes sont déterminés sur la base des taux d'ARNm transcrits à partir des gènes.

**4.** Procédé selon la revendication 3, dans lequel les taux d'ARNm sont déterminés par un procédé d'hybridation ou un procédé d'amplification d'acide nucléique.

**5.** Procédé selon la revendication 4, dans lequel les taux d'ARNm sont déterminés par un procédé d'hybridation à base de puces à ADN.

**6.** Procédé selon l'une quelconque des revendications 1 à 5, dans lequel dans l'étape (2), les échantillons cancéreux à tester sont classés sur la base de mises en évidence pathologiques choisies dans le groupe constitué de la morphologie cellulaire, des états d'infiltration vers les tissus périphériques, de la sensibilité contre les médicaments et des états des métastases dans les ganglions lymphatiques.

**7.** Procédé de classification d'un cancer, **caractérisé en ce que** le cancer est classé avec les taux d'expression des gènes dans un échantillon à tester, le procédé comprenant les étapes suivantes :

(a) la détermination des taux d'expression d'au moins un des gènes utilisés comme indices de classification des cancers, où les gènes sont choisis par le procédé de sélection selon l'une quelconque des revendications 1 à 6, dans l'échantillon à tester ; et
(b) la comparaison des taux d'expression des gènes déterminés dans l'étape (a) avec des taux d'expression des mêmes gènes dans un échantillon contrôle, classant de cette manière le cancer pour l'échantillon à tester.

**8.** Procédé selon la revendication 7, dans lequel dans l'étape (a), les taux d'expression d'au moins 5 types de gènes sont déterminés.

**9.** Procédé selon la revendication 7 ou 8, dans lequel les taux d'expression de gènes sont déterminés sur la base des taux d'ARNm transcrits à partir du gène, ou des taux des polypeptides traduits à partir des gènes.

**10.** Procédé selon la revendication 9, dans lequel les taux des ARNm sont déterminés par un procédé d'hybridation ou un procédé d'amplification d'acide nucléique.

**11.** Procédé selon la revendication 10, dans lequel les taux des ARNm sont déterminés par un procédé d'hybridation à base de micropuces à ADN.

**12.** Procédé selon la revendication 9, dans lequel les taux des polypeptides sont déterminés en utilisant des anticorps capables de se lier spécifiquement aux polypeptides ou à des fragments de ceux-ci.

**13.** Procédé selon l'une quelconque des revendications 8 à 12, dans lequel les taux d'expression d'au moins 5 types de gènes choisis dans le Groupe 1 suivant et/ou les taux d'expression d'au moins 5 types de gènes choisis dans le Groupe II suivant sont déterminés :

Groupe I :

- gène de la protéine 2 de liaison au facteur de croissance de type insulinique (IGFBP2) (GenBank, numéro d'accession : X16302) ;
- gène BIGH3 (GenBank, numéro d'accession : M77349) ;
- gène de la protéine 6 de liaison au facteur de croissance de type insulinique (IGFBP6) (GenBank, numéro d'accession : M62402) ;
- gène de la gélatinase A (MMP-2), (GenBank, numéro d'accession : M55593) ;
- gène de la kératine du cytosquelette 7 de type II (cytokératine 7 (K7 ; CK 7)), (GenBank, numéro d'accession : M13955) ;
- gène de la desmoplakine I, (GenBank, numéro d'accession : M77830) ;
- gène de la glutathion-S-transférase A1, (GenBank, numéro d'accession : M16594) ;
- gène de la glutathion-S-transférase Pi (GSTP1), (GenBank, numéro d'accession: U12472) ;
- gène de la collagénase 3 (MMP-13), (GenBank, numéro d'accession : X75308);

- gène de la kératine du cytosquelette 5 de type II (cytokératine 5 (K5; CK 5)), (GenBank, numéro d'accession : M21389) ;
- gène de la cadhérine P, (GenBank, numéro d'accession : X63629) ;
- gène de la kératine du cytosquelette 14 de type I (cytokératine 14 (K14 ; CK 14)), (GenBank, numéro d'accession : J00124) ;
- gène de la kératine du cytosquelette 6 de type II (cytokératine 6B (CK 6B)), (GenBank, numéro d'accession : L42610) ;
- gène de la matrilysine (MMP-7), (GenBank, numéro d'accession : X07819) ;
- gène "forkhead-like » 7, (GenBank, numéro d'accession : AF048693) ;
- gène du facteur de croissance du tissu conjonctif (CTGF), (GenBank, numéro d'accession : M92934) ;
- gène de la protéine de liaison au facteur de croissance de type insulinique dépendant de l'hormone de croissance, (GenBank, numéro d'accession : M35878) ; et
- gène de la protéine Rho8, (GenBank, numéro d'accession : X95282) ;

Groupe II :

- gène RBA/p48, (GenBank, numéro d'accession : X74262) ;
- gène de l'homologue de la protéine 2 de contrôle de la division cellulaire (EC 2.7.1.-) (cdc2), (GenBank, numéro d'accession : X05360) ;
- gène de la sous-unité de 38 kDa du facteur de réplication C (RFC38), (GenBank, numéro d'accession : L07541) ;
- gène du précurseur de l'apopaïne, (GenBank, numéro d'accession : U13737) ;
- gène de la protéine p58/HHR23B de complémentation de la réparation de xeroderma pigmentosum du groupe C, (GenBank, numéro d'accession : D21090) ;
- gène de la cycline G2, (GenBank, numéro d'accession : U47414) ;
- gène de la cycline A, (GenBank, numéro d'accession : X51688) ;
- gène de la protéine associée à l'apoptose TFAR15, (GenBank, numéro d'accession : AF022385) ;
- gène du récepteur de la tyrosine kinase TRKB, (GenBank, numéro d'accession : U12140) ;
- gène pour le transducteur de signaux et l'activateur de transcription 1-alpha/bêta (STAT1), (GenBank, numéro d'accession : M97935) ;
- oncogène K-ras, (GenBank, numéro d'accession : M54968) ;
- gène de la protéine de la susceptibilité du rétinoblastome (RB1), (GenBank, numéro d'accession : L41870) ;
- gène de la protéine d'interaction 1 de 19 kD de BCL2/adénovirus E1B (BNIP1), ARNm, cds complet, (GenBank, numéro d'accession : AF083957) ; et
- gène de l'inhibiteur de la protéine 1 de l'apoptose (HIAP-1), (GenBank, numéro d'accession : U45878).

**14.** Procédé selon l'une quelconque des revendications 7 à 13, dans lequel un échantillon à tester est classé sur la base du risque de métastases dans les ganglions lymphatiques.

**15.** Procédé de détection d'un cancer, comprenant les étapes suivantes :

(1) la détermination des taux d'expression d'au moins un des gènes utilisés comme indices de classification des cancers dans un échantillon à tester, où les gènes sont sélectionnés par le procédé de sélection selon l'une quelconque des revendications 1 à 6, et
(2) la comparaison des taux d'expression de gènes dans l'échantillon à tester déterminés dans l'étape (1) avec les taux d'expression des gènes dans un échantillon contrôle, détectant de cette manière un cancer,

où des expressions des acides nucléiques correspondant à au moins un des gènes ou des expressions des polypeptides codés par au moins un des gènes utilisés comme indices de classification des cancers sont modifiées par comparaison à un échantillon contrôle, sont un indice de la présence de cellules cancéreuses dans l'échantillon à tester.

**16.** Procédé selon la revendication 15, dans lequel dans l'étape (1), les taux d'expression d'au moins 5 types de gènes sont déterminés.

**17.** Procédé selon la revendication 15 ou 16, dans lequel les taux d'expression de gènes sont déterminés sur la base des taux d'ARNm transcrits à partir des gènes, ou des taux de polypeptides traduits à partir des gènes.

**18.** Procédé selon la revendication 17, dans lequel les taux d'ARNm sont déterminés par un procédé d'hybridation ou un procédé d'amplification d'acide nucléique.

**19.** Procédé selon la revendication 18, dans lequel les taux d'ARNm sont déterminés par un procédé d'hybridation à base de micropuces à ADN.

**20.** Procédé selon la revendication 17, dans lequel les taux des polypeptides sont déterminés en utilisant des anticorps capables de se lier spécifiquement aux polypeptides ou à des fragments de ceux-ci.

**21.** Procédé selon l'une quelconque des revendications 15 à 20, dans lequel les taux d'expression d'au moins 5 types de gènes choisis parmi les gènes du Groupe I selon la revendication 13 et/ou les taux d'expression d'au moins 5 types de gènes choisis parmi les gènes du groupe II selon la revendication 13, sont déterminés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000219807 A


### Non-patent literature cited in the description


- **FEARON, E.R. et al.** *Cell,* 1990, vol. 61, 759-767
- **SUGIMURA, T.** *Science,* 1992, vol. 258, 603-607
- Molecular Cloning, A laboratory manual. 1989, 9.52-9.55
- Current Protocols in Immunology. John Wiely & Sons, Inc, 1992
- **LINDA G. LEE et al.** *Nucleic Acids Research,* 1993, vol. 21, 3761-3766